# EUROPEAN PATENT APPLICATION

(11) **EP 4 578 404 A1**
(43) Date of publication of application: **02.07.2025**
(21) Application number: 24223277.5
(22) Date of filing: 25.12.2024
(51) Int. Cl.: A61B 8/08, A61B 8/12, A61B 8/00

(54) **ULTRASOUND PROBE**

(30) Priority: 26.12.2023 CN 202311827795; 26.12.2023 CN 202311817302
(71) Applicant: Wuhan United Imaging Healthcare Co., Ltd., WuHan, Hubei 430206 (CN)
(72) Inventor: SONG, Wei, Wuhan, 430206 (CN); YAO, Yuan, Wuhan, 430206 (CN); ZHU, Zhihao, Wuhan, 430206 (CN); SI, Kang, Wuhan, 430206 (CN)
(74) Representative: Wang, Bo

(57) **Abstract**

The present disclosure provides an ultrasound probe. The ultrasound probe may include an ultrasonic transducer and a conduit mechanism including a flexible conduit and at least one first connecting member. The flexible conduit may be connected to the ultrasonic transducer, and the at least one first connecting member may be connected to the flexible conduit. The ultrasound probe may also include a handle mechanism including at least one second connecting member. The handle mechanism may be in a connected state or a disconnected state. In the connected state, the handle mechanism may be connected with the conduit mechanism and in the disconnected state, the handle mechanism may be separated from the conduit mechanism. Each of the at least one second connecting member may be detachably connected with one of the at least one first connecting member to switch the handle mechanism between the connected state and the disconnected state.

## Description

### CROSS-REFERENCE TO RELATED APPLICATIONS

This application claims priority to Chinese application No. 202311817302.3 filed on December 26, 2023, and Chinese application No. 202311827795.9 filed on December 26, 2023, the entire contents of which are incorporated herein by reference.

### TECHNICAL FIELD

The present disclosure relates to the technical field of medical devices, and in particular, to ultrasound probes.

### BACKGROUND

Intracardiac Echocardiography (ICE) refers to a diagnostic technique using a miniature ultrasonic transducer that is installed at the tip of a flexible conduit and is sent to a heart chamber through peripheral blood vessels (veins). The miniature ultrasonic transducer emits sound waves, echoes generated by the tissue of a subject that receives the sound waves and an ultrasound image may be formed by processing the received echo through a computer, which provides high-resolution, real-time images of an anatomical structure of the heart chambers to monitor the hemodynamic status in real time. Usually, an intracardiac ultrasound probe may include a flexible conduit and a handle mechanism connected to a proximal end of the flexible conduit. The direction of a distal end of the flexible conduit may be adjusted by controlling the handle mechanism, so that the ultrasonic transducer may be directed toward different regions. However, an intracardiac ultrasound device needs to be provided with too many components for adjusting the direction of the distal end of the flexible conduit, and the structure of the intracardiac ultrasound device is relatively complicated, which results in a relatively high assembly difficulty, a relatively high manufacturing cost, and a relatively high use cost.

Therefore, it is desirable to provide an ultrasound probe. At least a portion of components of the ultrasound probe may be reused to reduce the cost of use, and simplify the structure of the ultrasound probe required to adjust the direction of the distal end of the flexible conduit to reduce the assembly difficulty and the manufacture cost.

### SUMMARY

One of the embodiments of the present disclosure provides an ultrasound probe. The ultrasound probe may include: an ultrasonic transducer; a conduit mechanism including a flexible conduit and at least one first connecting member, the flexible conduit being connected to the ultrasonic transducer, and the at least one first connecting member being connected to the flexible conduit; and a handle mechanism including at least one second connecting member, the handle mechanism having a connected state or a disconnected state, in the connected state, the handle mechanism being connected with the conduit mechanism and in the disconnected state, the handle mechanism being separated from the conduit mechanism. Each of the at least one second connecting member may be detachably connected with one of the at least one first connecting member to switch the handle mechanism between the connected state and the disconnected state.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 is a schematic diagram illustrating an exemplary ultrasound probe according to some embodiments of the present disclosure;
FIG. 2 is a schematic diagram illustrating an ultrasound probe according to some embodiments of the present disclosure;
FIG. 3 is a schematic diagram illustrating a first connecting member and a second connecting member according to some embodiments of the present disclosure;
FIG. 4 is schematic diagram illustrating a cross section of a distal end of a flexible conduit being perpendicular to an axial direction of flexible conduit according to some embodiments of the present disclosure;
FIG. 5A is another schematic diagram illustrating a first connecting member and a second connecting member according to some embodiments of the present disclosure;
FIG. 5B is a schematic diagram illustrating a portion of a second connecting member according to some embodiments of the present disclosure;
FIG. 6A is a partially enlarged view of a connection between a first connecting member and a second connecting member according to some embodiments of the present disclosure;
FIG. 6B is another partially enlarged view of a connection between a first connecting member and a second connecting member according to some embodiments of the present disclosure;
FIG. 7 is a schematic diagram illustrating a first connecting member according to some embodiments of the present disclosure;
FIG. 8 is a schematic diagram illustrating a second connecting member according to some embodiments of the present disclosure;
FIG. 9 is another schematic diagram illustrating a first connecting member and a second connecting member according to some embodiments of the present disclosure;
FIG. 10 is another schematic diagram illustrating a first connecting member and a second connecting member according to some embodiments of the present disclosure;
FIG. 11 is a schematic diagram illustrating some components of a conduit mechanism and a handle mechanism according to some embodiments of the present disclosure;
FIG. 12 is a schematic diagram illustrating a first gear ring a first gear, and a second connecting member according to some embodiments of the present disclosure;
FIG. 13 is a partially enlarged view of a connection of a first gear ring, a first gear, and a second connecting member according to some embodiments of the present disclosure;
FIG. 14 is a schematic diagram illustrating a first gear ring and a first gear according to some embodiments of the present disclosure;
FIG. 15 is another schematic diagram illustrating a first gear ring and a first gear according to some embodiments of the present disclosure;
FIG. 16 is a schematic diagram illustrating a first friction wheel and a second friction wheel according to some embodiments of the present disclosure;
FIG. 17 is a schematic diagram illustrating a first gear ring, a first connecting member, and a second connecting member according to some embodiments of the present disclosure;
FIG. 18 is a schematic diagram illustrating a second gear ring, a second gear, and a second connecting member according to some embodiments of the present disclosure;
FIG. 19 is a schematic diagram illustrating a first gear, a second gear, and a second base according to some embodiments of the present disclosure;
FIG. 20 is another schematic diagram illustrating a second gear ring, a second gear, and a second connecting member according to some embodiments of the present disclosure;
FIG. 21 is a schematic diagram illustrating a plurality of second gears according to some embodiments of the present disclosure;
FIG. 22 is a schematic diagram illustrating a third gear ring, a first connecting member, and a second connecting member according to some embodiments of the present disclosure;
FIG. 23 is a schematic diagram illustrating an ultrasound probe according to some embodiments of the present disclosure;
FIG. 24 is a schematic diagram illustrating a flexible conduit in different bending directions according to some embodiments of the present disclosure;
FIG. 25 is a schematic diagram illustrating a portion of a handle mechanism according to some embodiments of the present disclosure;
FIG. 26 is a schematic diagram illustrating a frame, a first rotating rod, and a second rotating rod according to some embodiments of the present disclosure;
FIG. 27 is a schematic diagram illustrating a frame, a sliding member, a first bend-adjusting wire, and a second bend-adjusting wire according to some embodiments of the present disclosure;
FIG. 28 is another schematic diagram illustrating a portion of a handle mechanism according to some embodiments of the present disclosure;
FIG. 29 is a schematic diagram illustrating a first rotating rod, a second rotating rod, a third gear, a fourth gear, and a fourth gear ring according to some embodiments of the present disclosure;
FIG. 30 is a schematic diagram illustrating a frame according to some embodiments of the present disclosure;
FIG. 31 is a schematic diagram illustrating a locking assembly according to some embodiments of the present disclosure;
FIG. 32 is another schematic diagram illustrating a portion of a handle mechanism according to some embodiments of the present disclosure;
FIG. 33 is a schematic diagram illustrating a rotating rod, a first support, a third gear, a fourth gear, and a fourth gear ring according to some embodiments of the present disclosure;
FIG. 34 is another schematic diagram illustrating a portion of a handle mechanism according to some embodiments of the present disclosure;
FIG. 35 is another schematic diagram illustrating a locking assembly according to some embodiments of the present disclosure;
FIG. 36 is a schematic diagram illustrating a limiting sleeve according to some embodiments of the present disclosure;
FIG. 37 is a schematic diagram illustrating a sawtooth member according to some embodiments of the present disclosure;
FIG. 38 is a schematic diagram illustrating a moving member according to some embodiments of the present disclosure; and
FIG. 39 is another schematic diagram illustrating a locking assembly according to some embodiments of the present disclosure.

### DETAILED DESCRIPTION

In order to more clearly illustrate the technical solutions relating to the embodiments of the present disclosure, a brief introduction of the drawings referred to the description of the embodiments is provided below. Obviously, the drawings described below are only some examples or embodiments of the present disclosure. Those having ordinary skills in the art, without further creative efforts, may apply the present disclosure to other similar scenarios according to these drawings. Unless obviously obtained from the context or the context illustrates otherwise, the same numeral in the drawings refers to the same structure or operation.

It should be understood that the "system," "device," "unit," and/or "module" used herein are one method to distinguish different components, elements, parts, sections, or assemblies of different levels. However, if other words may achieve the same purpose, the words may be replaced by other expressions.

It should be noted that the terms "proximal end" and "distal end" are used in the present disclosure to indicate an orientation, which may be configured to indicate a direction in which a device (e.g., an ultrasound probe) or a component enters the human body. An end of the device or the component facing an operator (e.g., a physician, a nurse, etc.) before the device or component is ready to enter the human body is the "proximal end", and an end that extends into the human body to perform the treatment is the "distal end", and furthermore, "proximal" and "distal" are used to indicate orientation. In addition, the terms "proximal end" and "distal end" shall not be understood as denoting ends only.

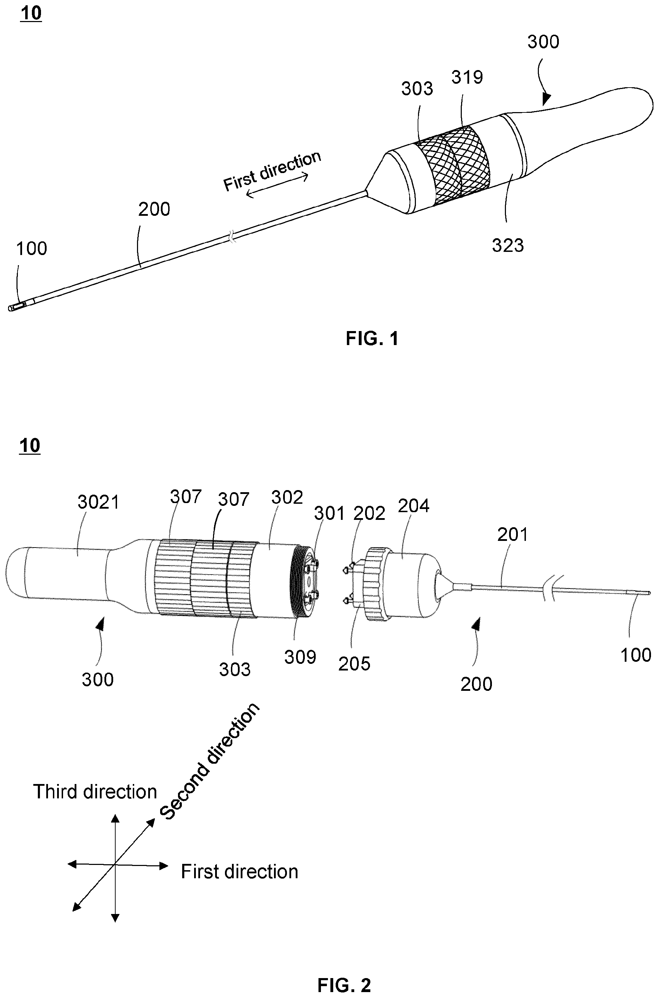

FIG. 1 is a schematic diagram illustrating an exemplary ultrasound probe according to some embodiments of the present disclosure.

The ultrasound probe 10 may be configured to convert an electrical signal emitted by an ultrasound imaging system into an ultrasound wave used to probe a region (e.g., the heart) of a patient, receive a reflected signal of the ultrasound wave from the region, and convert the reflected signal back to an electrical signal, and transmit the electrical signal to the ultrasound imaging system, thereby realizing ultrasound imaging. In some embodiments, as shown in FIG. 1, the ultrasound probe 10 may include an ultrasonic transducer 100, a conduit mechanism 200, and a handle mechanism 300.

The ultrasonic transducer 100 may be configured to convert the received electrical signal emitted by the ultrasound imaging system into the ultrasound wave. The ultrasound wave emitted by the ultrasonic transducer 100 used to probe the region (e.g., the heart) of the patient. The ultrasonic transducer 100 may receive the reflected signal of the ultrasound wave, convert the reflected signal into the electrical signal, and transmit the electrical signal to the ultrasound imaging system.

The ultrasonic transducer 100 may be disposed at the distal end of the conduit mechanism 200 and located at the distal end of the ultrasound probe 10. In some embodiments, the ultrasonic transducer 100 may be disposed at the distal end of the conduit mechanism 200 in various ways. For example, the ultrasonic transducer 100 may be disposed at the distal end of the conduit mechanism 200 by hot melt welding. As another example, the ultrasonic transducer 100 may be bonded to the distal end of the conduit mechanism 200.

The conduit mechanism 200 may be configured to connect the ultrasonic transducer 100 with the handle mechanism 300.

In some embodiments, the conduit mechanism 200 may include a flexible conduit 201 and at least one first connecting member 202.

The flexible conduit 201 may connect with the ultrasonic transducer 100. FIG. 2 is a schematic diagram illustrating an ultrasound probe according to some embodiments of the present disclosure. As shown in FIG. 2, the ultrasonic transducer 100 may be disposed at the distal end of the flexible conduit 201. The flexible conduit 201 may be a hollow pipe. A circuit board (e.g., a flexible circuit board), cables, and other components of the ultrasonic transducer 100 may be disposed in the hollow pipe of the flexible conduit 201 and connected to the ultrasound imaging system.

In some embodiments, each of the at least one first connecting member 202 may be connected with the flexible conduit 201.

The handle mechanism 300 may be configured for an operator to operate the ultrasound probe 10.

In some embodiments, the handle mechanism 300 may include at least one second connecting member 301. Each of the at least one first connecting member 202 may be detachably connected with one of the at least one second connecting member 301 to switch the handle mechanism 300 between a connected state and a disconnected state. The connected state refers to a state in which the handle mechanism 300 is connected with the conduit mechanism 200. The disconnected state refers to a state in which the handle mechanism 300 is separated from the conduit mechanism 200.

For example, one of the at least one first connecting member 202 may be detachably connected with one of the at least one second connecting member 301 via plug connection, thread connection, or the like, or any combination thereof to switch the handle mechanism 300 between the connected state and the disconnected state. More descriptions regarding the one of the at least one first connecting member 202 being detachably connected with one of the at least one second connecting member 301 may be found below.

In some embodiments, after one of the at least one first connecting member 202 is detachably connected with one of the at least one second connecting member 301, the handle mechanism 300 may be detachably connected with other components (e.g., a second housing 302 of the handle mechanism 300, a first housing 204 in the conduit mechanism 200) of the conduit mechanism 200 to ensure the stability of the connection between the one of the at least one first connecting member 202 and the one of the at least one second connecting member 301, and improve the connection strength and connection stability of the conduit mechanism 200 and the handle mechanism 300 in the connected state, so that the conduit mechanism 200 and the handle mechanism 300 may not be easily separated. More descriptions regarding the embodiments may be found below.

In some embodiments of the present disclosure, by switching the handle mechanism 300 between the connected state and the disconnected state, the conduit mechanism 200 connected with the handle mechanism 300 may be replaced, so that the handle mechanism 300 may be used repeatedly, thereby reducing the use cost of the ultrasound probe 10 and reducing waste of resources.

In some embodiments, the conduit mechanism 200 may include at least one bend-adjusting wire 203. The at least one bend-adjusting wire 203 may also be disposed in the hollow pipe of the flexible conduit 201. The proximal end of one of the at least one bend-adjusting wire 203 may be connected to one of the at least one first connecting member 202. The distal end of the one of the at least one bend-adjusting wire 203 may be connected to the inner wall of the distal end of the flexible conduit 201, so that the at least one first connecting member 202 may be connected to the flexible conduit 201. For example, the inner wall of the distal end of the flexible conduit 201 may be connected with a metal ring, and the distal end of each of the at least one bend-adjusting wire 203 may extend into the flexible conduit 201 and is fixed to the metal ring. In this way, when each of the at least one first connecting member 202 moves in a first direction, the one of the at least one bend-adjusting wire 203 connected to each first connecting member 202 may be driven to move, so that the distal end of the flexible conduit 201 may be bent, and an orientation of the ultrasonic transducer 100 at the distal end of the flexible conduit 201 may be adjusted. As shown in FIG. 2, the first direction described herein is a direction parallel to the length direction of the handle mechanism 300. The second direction, the third direction, and the first direction may be perpendicular to each other.

In some embodiments, the count of the at least one bend-adjusting wire 203 may be 1. Accordingly, a count of the at least one first connecting member 202 may be 1, and a count of the at least one second connecting member 301 may be also 1. The second connecting member 301 may be detachably connected with the first connecting member 202, and the first connecting member 202 may be connected with the bend-adjusting wire 203. When the second connecting member 301 moves in the first direction, the first connecting member 202 and the bend-adjusting wire 203 may be driven to move in the first direction. More descriptions regarding controlling the second connecting member 301 to move in the first direction may be found below. When the bend-adjusting wire 203 moves in the first direction, the distal end of the flexible conduit 201 may be driven to bend, so that the orientation of the ultrasonic transducer 100 disposed at the distal end of the flexible conduit 201 may be adjusted. More descriptions regarding driving the bend-adjusting wire 203 to move in the first direction may be found below.

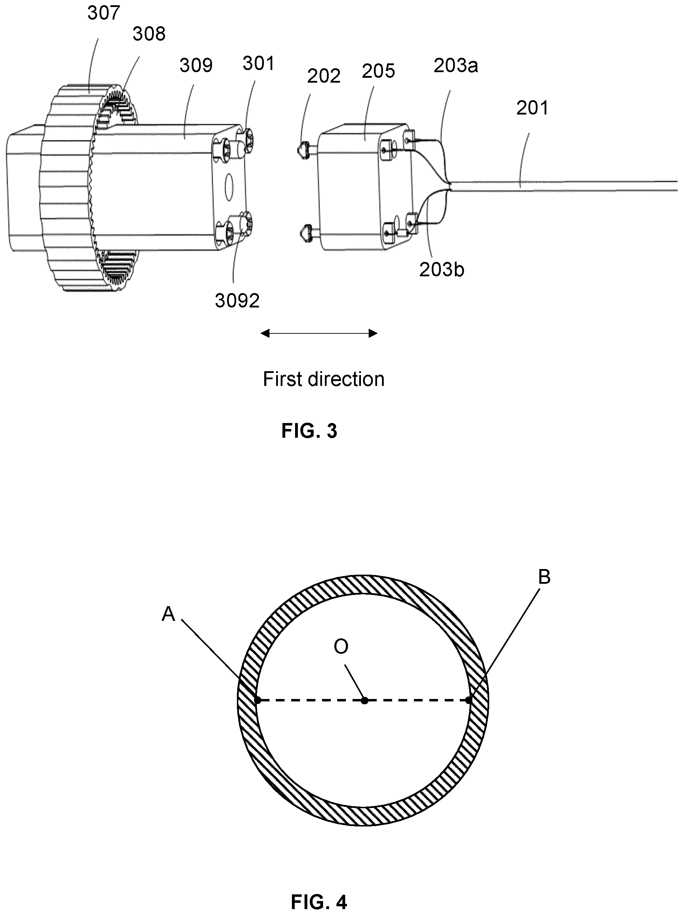

In some embodiments, the at least one bend-adjusting wire 203 may be divided into at least one group. Each group of the at least one group of bend-adjusting wires may include a first bend-adjusting wire 203a and a second bend-adjusting wire 203b. One end of the first bend-adjusting wire 203a and one end of the second bend-adjusting wire 203b may be respectively connected to the inner wall of a distal end of the flexible conduit 201. Each of the other end of the first bend-adjusting wire 203a and the other end of the second bend-adjusting wire 203b may be connected with one of two first connecting members 202 among the at least one first connecting member 202. In the connected state, each of the two first connecting members 202 may be connected with one of two second connecting members 301 among the at least one second connecting member 301. The first bend-adjusting wire 203a and the second bend-adjusting wire 203b may be connected to different positions of the inner wall of the distal end of the flexible conduit 201. In some embodiments, the first bend-adjusting wire 203a and the second bend-adjusting wire 203b may be connected to a position opposite to the inner wall of the distal end of the flexible conduit 201, so that the operator may control a direction of the distal end of the flexible conduit 201. For example, as shown in FIG. 4, the first bend-adjusting wire 203a and the second bend-adjusting wire 203b may be connected to point A and point B of the inner wall of the distal end of the flexible conduit 201, respectively. A connection line between point A and point B passes through the central point O of positions corresponding to point A and point B of the flexible conduit 201. Accordingly, when the first bend-adjusting wire 203a and the second bend-adjusting wire 203b move in opposite directions along the first direction, one of the first bend-adjusting wire 203a and the second bend-adjusting wire 203b may pull the distal end of the flexible conduit 201 to move toward the proximal end of the flexible conduit 201, and the other of the first bend-adjusting wire 203a and the second bend-adjusting wire 203b may push the distal end of the flexible conduit 201 to move away from the proximal end of the flexible conduit 201, which may make the distal end of the flexible conduit 201 easier to be bent in a desired bending direction, with less difficulty in bending.

In some embodiments, the count of groups of the at least one group of bend-adjusting wires 203 may be 1. Accordingly, the count of the at least one first connecting member 202 may be 2, and the count of the at least one second connecting member 301 may be also 2. One second connecting member 301 of the two second connecting members 301 may be detachably connected to one first connecting member 202 of the two first connecting members 202. The first connecting member 202 may be connected to the first bend-adjusting wire 203a of the group of bend-adjusting wires. The other second connecting member 301 of the two second connecting members 301 may be detachably connected to the other first connecting member 202 of the two first connecting members 202. The other first connecting member 202 may be connected to the second bend-adjusting wire 203b of the group of bend-adjusting wires. When the two second connecting members 301 move in the first direction, the two first connecting members 202 may be driven to move in the first direction, respectively, so that the first bend-adjusting wire 203a and the second bend-adjusting wire 203b may be driven to move in the first direction. The two second connecting members 301 may move in opposite directions in the first direction. Accordingly, the first bend-adjusting wire 203a and the second bend-adjusting wire 203b may move in opposite directions in the first direction.

In some embodiments, the count of groups of the at least one group of bend-adjusting wires may be greater than 1. In other words, the conduit mechanism 200 may include multiple groups of bend-adjusting wires. A connection line between the distal end of the first bend-adjusting wire 203a and the distal end of the second bend-adjusting wire 203b in each group may intersect with a connection line between a distal end of the first bend-adjusting wire 203a and a distal end of the second bend-adjusting wire 203b in another group among the multiple groups of bend-adjusting wires. FIG. 3 is a schematic diagram illustrating a first connecting member and a second connecting member according to some embodiments of the present disclosure. As shown in FIG. 3, the conduit mechanism 200 may include multiple groups of bend-adjusting wires 203. Each of the first bend-adjusting wire 203a and the second bend-adjusting wire 203b in each group may be connected to one of the at least one first connecting member 202. The handle mechanism 300 may include multiple second connecting members 301 and multiple first connecting members 202 and each of the multiple second connecting members 301 may be connected to one of the multiple first connecting members 202. The conduit mechanism 200 may include the multiple groups of bend-adjusting wires 203, and when the multiple groups of bend-adjusting wires 203 are connected with the inner wall of the distal end of the flexible conduit 201 at a same position along the first direction, for example, when the multiple groups of bend-adjusting wires 203 are connected with a same metal ring of the inner wall of the distal end of the flexible conduit 201, each connection line between the distal end of the first bend-adjusting wire 203a and the distal end of the second bend-adjusting wire 203b in each of the multiple groups of bend-adjusting wires 203 may intersect at a point (e.g., a center of the metal ring), so that the distal end of the flexible conduit 201 may be bent in various orientations, and the ultrasonic transducer 100 can have more orientations and be used more flexibly.

As shown in FIG. 3, in the embodiment, the conduit mechanism 200 may include two groups of bend-adjusting wires 203 and the connection lines each of which is between the distal end of the first bend-adjusting wire 203a and the distal end of the second bend-adjusting wire 203b in one group of the two groups of bend-adjusting wires 203 may be perpendicular, so that the distal end of the flexible conduit 201 may be bent in four directions. For example, two bending wires in one group of bending wires 203 may control the distal end of the flexible conduit 201 to bend upwards and downward along the third direction, respectively, and two bending wires in the other group of bending wires 203 may control the distal end of the flexible conduit 201 to bend to the left and to the right along the second direction, respectively. In other embodiments, the conduit mechanism 200 may include a larger count of groups of bend-adjusting wires, and a structure of each group of bend-adjusting wires 203 may be the same or different.

More descriptions regarding driving each group of bend-adjusting wires 203 to bend the distal end of the flexible conduit 201 may be found elsewhere in the present disclosure.

In some embodiments, one of the at least one first connecting member 202 may be detachably connected to one of the at least one second connecting member 301 in various ways. For example, in the connected state, a second connecting member 301 may restrict a position of a first connecting member 202 in a first direction, so that the first connecting member 202 may move with the second connecting member 301 in the first direction. When the first connecting member 202 needs to be disconnected from the second connecting member 301, the restriction of the second connecting member 301 on the position of the first connecting member 202 in the first direction may be released by rotating the first connecting member 202 and/or the second connecting member 301, thereby realizing the detachable connection. As another example, one of the at least one second connecting member 301 and one of the at least one first connecting member 202 may be detachably connected via a thread structure. As yet another example, one of the at least one second connecting member 301 and one of the at least one first connecting member 202 may be detachably connected via a plug structure. More descriptions regarding the embodiments may be found below.

In some embodiments, one of the at least one second connecting member 301 of the handle mechanism 300 may be detachably connected with one of the at least one first connecting member 202 of the conduit mechanism 200. In this way, an operator may control the second connecting member 301 via the handle mechanism 300, so as to control the at least one bend-adjusting wire 203, and control the orientation of the distal end of the flexible conduit 201 of the conduit mechanism 200, so that the orientation of the ultrasonic transducer 100 disposed at the distal end of the flexible conduit 201 may be adjusted without the need to an additional structure specializing in the adjustment of the orientation of the ultrasonic transducer 100, which can reduce a count of components, simplify the structure, and further reduce the cost. More descriptions regarding the controlling the second connecting member 301 to control the at least one bend-adjusting wire 203 may be found below.

How the first connecting member 202 and the second connecting member 301 are configured to realize the detachable connection between the first connecting member 202 and the second connecting member 301 is illustrated as follows.

In some embodiments, a first connecting member 202 and a second connecting member 301 that are detachably connected among the at least one first connecting member 202 and the at least one second connecting member 301 may be considered to be a group of connecting members.

In some embodiments, the ultrasound probe 10 may include only one group of connecting members. For example, the ultrasound probe 10 may be provided with one first connecting member 202, and one second connecting member 301, and the first connecting member 202 may be detachably connected to the second connecting member 301.

In some embodiments, the ultrasound probe 10 may include multiple groups of connecting members. Each group of the multiple groups of connecting members may include one first connecting member 202 and one second connecting member 301 that are detachably connected. One group of the first connecting member 202 and the second connecting member 301 that are detachably connected is described below in the present disclosure.

In some embodiments, the second connecting member 301 and the first connecting member 202 may be detachably connected via a plug connection. The proximal end of the first connecting member 202 may include a first plug member, and the distal end of the second connecting member 301 may include a second plug member. The first plug member may be fitted with the second plug member via a plug connection, so that the second connecting member 301 may be detachably connected to the first connecting member 202.

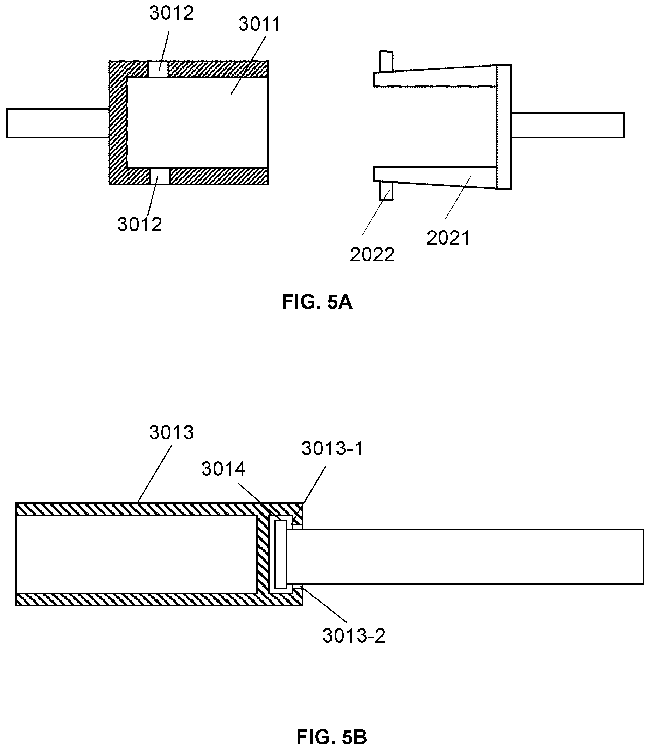

FIG. 5A is another schematic diagram illustrating a first connecting member and a second connecting member according to some embodiments of the present disclosure. As shown in FIG. 5A, the first plug member may be an inserter. The inserter may include two support bars 2021. Each of the two support bars 2021 may be provided with a limiting block 2022. The second plug member may be a connector. The connector may include a hollow channel 3011, and the hollow channel 3011 may include two jacks 3012. When the second connecting member 301 needs to be connected to the first connecting member 202, an operator may control the two support bars 2021 in the inserter to be deformed, such that a distance between distal ends of the two support bars 2021 may become smaller, and the two support bars 2021 may enter into the hollow channel 3011. The operator may control the first connecting member 202 to move toward a proximal end in a first direction until the limiting blocks 2022 on the two support bars 2021 enter into the jacks 3012. At this time, the jacks 3012 may limit the positions of the limiting blocks 2022, so that the second connecting member 301 may be connected to the first connecting member 202. When the connected second connecting member 301 and the first connecting member 202 need to be separated, the operator may control the two limiting blocks 2022 in the inserter to move toward each other, such that the distance between the two limiting blocks 2022 may become smaller. At the same time, the operator may control the first connecting member 202 to move toward the distal end in the first direction. At this time, the limiting blocks 2022 may leave the jacks 3012, and the jacks 3012 may relieve the position restriction on the limiting blocks 2022, so that the second connecting member 301 may be separated from the first connecting member 202.

In some embodiments, the second connecting member 301 may be fitted with the first connecting member 202 via other structures. For example, the second connecting member 301 may be fitted with the first connecting member 202 via a pin structure. The first connecting member 202 may be one of a pin hole or a pin, and the second connecting member 301 may be the other of the pin hole or the pin.

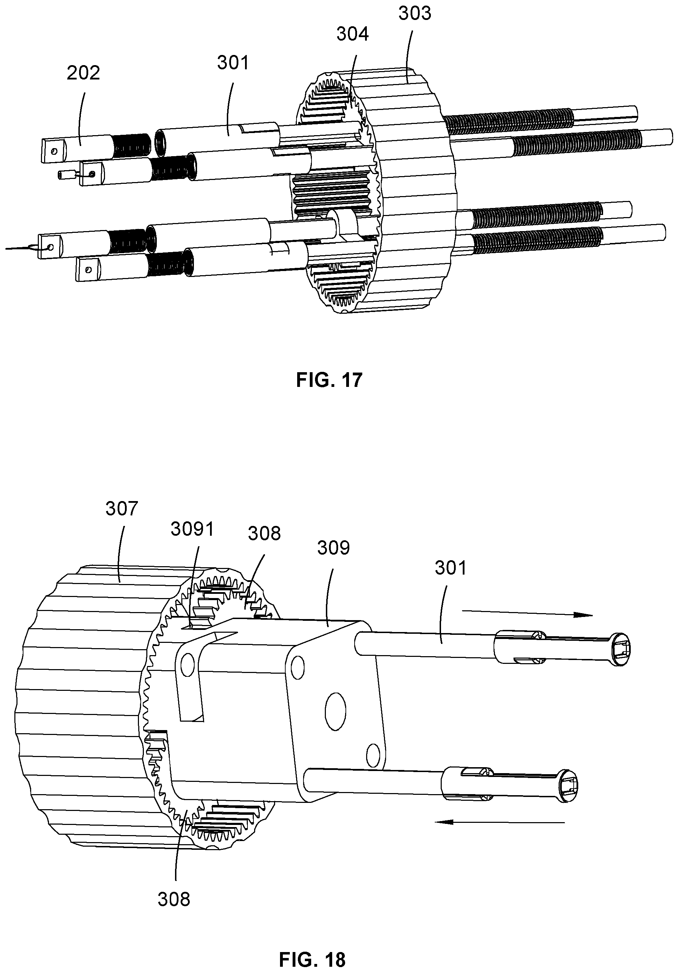

In some embodiments, the second connecting member 301 may be detachably connected to the first connecting member 202 via a thread connection. For example, FIG. 17 is a schematic diagram illustrating a first gear ring, a first connecting member, and a second connecting member according to some embodiments of the present disclosure. As shown in FIG. 17, the proximal end of the first connecting member 202 may be provided with an external thread, the distal end of the second connecting member 301 may be provided with an internal thread, and the external thread and the internal thread may be adapted to each other, so that the second connecting member 301 and the first connecting member 202 may be detachably connected. As another example, the proximal end of the first connecting member 202 may be provided with an internal thread, and the distal end of the second connecting member 301 may be provided with an external thread. The external thread and the internal thread may be adapted, so that the second connecting member 301 and the first connecting member 202 may be detachably connected.

In some embodiments, when the second connecting member 301 is connected to the first connecting member 202 via the thread connection, a thread structure 3013 in the second connecting member 301 provided at the distal end of the second connecting member 301 may rotate relative to other parts of the second connecting member 301.

FIG. 5B is a schematic diagram illustrating a portion of a second connecting member according to some embodiments of the present disclosure. As shown in FIG. 5B, the second connecting member 301 may be provided with the thread structure 3013, and the thread structure 3013 may be provided with an internal thread. The thread structure 3013 may also be provided with a limiting hole 3013-1 ( also be referred to as a first limiting hole 3013-1). The second connecting member 301 may also include a limiting member 3014 provided inside the first limiting hole 3013-1. The limiting member 3014 may rotate inside the first limiting hole 3013-1. The first limiting hole 3013-1 may include a through-hole 3013-2 in flow communication with the outside. Other structures (e.g., a plug-in portion) of the second connecting member 301 may be connected to the limiting member 3014 through the through-hole 3013-2. The size of the limiting member 3014 in one of a second direction and a third direction may be larger than a size of the through-hole 3013-2 in one of the second direction and the third direction. In this way, the other structures of the second connecting member 301 may not drive the thread structure 3013 to rotate when rotating relative to the thread structure 3013, so as to avoid loosening of the connection between the second connecting member 301 and the first connecting member 202 caused by the rotation of the other structures of the second connecting member 301 when the second connecting member 301 is connected to the first connecting member 202 via thread connection.

In some embodiments, when the second connecting member 301 is connected to the first connecting member 202 via the thread connection, another thread structure in the first connecting member 202 provided at the proximal end of the first connecting member 202 may rotate relative to the second connecting member 301.

In some embodiments, the second connecting member 301 and the first connecting member 202 may be configured to rotate relative to each other around an axis parallel to a first direction to enable switching of the handle mechanism 300 between a connected state and the disconnected state. For example, when the second connecting member 301 is connected to the first connecting member 202 via the thread connection, the thread structure 3013 in the second connecting member 301 may rotate relative to the thread structure 3013 in the first connecting member 202 to enable switching of the handle mechanism 300 between the connected state and the disconnected state. The thread structure 3013 in the second connecting member 301 may rotate relative to the second connecting member 301 to avoid influencing states of other parts of the second connecting member 301 or other structures connected to the second connecting member 301 when the thread structure 3013 in the second connecting member 301 rotates. As another example, when the second connecting member 301 is threadedly connected with the first connecting member 202, another thread structure in the first connecting member 202 may rotate relative to the second connecting member 301 to enable the switching of the handle mechanism 300 between the connected state and the disconnected state.

In some embodiments, one of the first connecting member 202 and the second connecting member 301 may include a slot 3015, and the other one of the first connecting member 202 and the second connecting member 301 may include an insert block 2023. The dimension of the slot 3015 along a second direction may be smaller than the dimension of the slot 3015 along a third direction.

In some embodiments, at least one of the slot 3015 or the insert block 2023 may be configured to rotate around an axis parallel to the first direction to switch the at least one of the slot 3015 or the insert block 2023 between a first state and a second state. In the first state, the dimension of the slot 3015 along one of the second direction and the third direction may be greater than the dimension of the insert block 2023 along the one of the second direction and the third direction. The insert block 2023 may be configured to be inserted into or withdrawn from the slot 3015 along the first direction. In the second state, the dimension of the slot 3015 in the second direction may be smaller than the dimension of the insert block 2023 in the second direction, or the dimension of the slot 3015 in the third direction may be smaller than the dimension of the insert block 2023 in the third direction to prevent the insert block 2023 being withdrawn from the slot 3015. The slot 3015 and the insert block 2023 may be non-circular in a cross-section perpendicular to the first direction. The first state may be illustrated as a base state. In the first state, the width direction of the slot 3015 may be parallel to the width direction of the insert block 2023, and the length direction of the slot 3015 may be parallel to the length direction of the insert block 2023. The dimension of the slot 3015 along each direction may be greater than the dimension of the insert block 2023 along a corresponding direction. For example, the dimension of the slot 3015 along the width direction may be greater than the dimension of the insert block 2023 along the width direction, and the dimension of the slot 3015 along the length direction may be greater than the dimension of the insert block 2023 along the length direction. Therefore, at this time, the dimension of the slot 3015 along the second direction may be greater than the dimension of the insert block 2023 along the second direction, and the dimension of the slot 3015 along the third direction may be greater than the dimension of the insert block 2023 along the third direction. The insert block 2023 may be configured to be inserted into or withdrawn from the slot 3015 along the first direction. In the second state, the width direction of the slot 3015 may be not parallel to the width direction of the insert block 2023, and the length direction of the slot 3015 may be not parallel to the length direction of the insert block 2023. At this time, the dimension of the slot 3015 in the second direction may be smaller than the dimension of the insert block 2023 in the second direction, and/or the dimension of the slot 3015 in the third direction may be smaller than the dimension of the insert block 2023 in the third direction to prevent the insert block 2023 from being withdrawn from the slot 3015.

The slot 3015 may be provided at the proximal end of the first connecting member 202 or the distal end of the second connecting member 301. When the slot 3015 is provided at the proximal end of the first connecting member 202, the insert block 2023 may be provided at the distal end of the second connecting member 301. When the slot 3015 is provided at the distal end of the second connecting member 301, the insert block 2023 may be provided at the proximal end of the first connecting member 202.

The first connecting member 202 and the second connecting member 301 may be both rod-shaped. The proximal end of the first connecting member 202 may be provided with the insert block 2023, and the distal end of the second connecting member 301 may be provided with the slot 3015. At least one of the slot 3015 or the insert block 2023 may be configured to rotate around an axis parallel to the first direction to achieve relative rotation of the slot 3015 and the insert block 2023 to switch the at least one of the slot 3015 or the insert block 2023 between the first state and the second state, so the handle mechanism 300 may be switched between the connected state and the disconnected state.

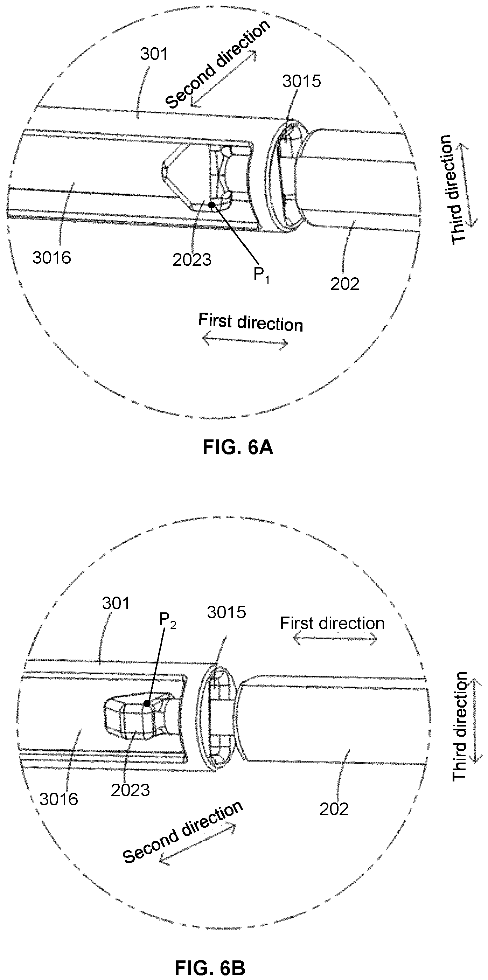

For example, as shown in FIG. 6A and FIG. 6B, the first connecting member 202 may rotate around an axis parallel to the first direction to switch the insert block 2023 between a first position shown in FIG. 6A and a second position shown in FIG. 6B. In the first state, the insert block 2023 may be in the first position. In the second state, the insert block 2023 may be in the second position. The first position and the second position may be different positions on a circumferential direction of the first connecting member 202 in the process of the first connecting member 202 rotating around an axis parallel to the first direction. For example, as shown in FIG. 6A, in the first state, a point in the insert block 2023 is located at position P1. As shown in FIG. 6B, in the second state, the point in the insert block 2023 is located at position P2. When the insert block 2023 is in the first position shown in FIG. 6A, the dimension of the slot 3015 along the second direction may be greater than the dimension of the insert block 2023 along the second direction, and the dimension of the slot 3015 along the third direction may be greater than the dimension of the insert block 2023 along the third direction, so that the insert block 2023 and the slot 3015 may be fitted and the insert block 2023 may be inserted into or withdrawn from the slot 3015 along the first direction. When the insert block 2023 is rotated to the second position shown in FIG. 6B, the dimension of the slot 3015 along the second direction may be smaller than the dimension of the insert block 2023 along the second direction, and the insert block 2023 inserted into the slot 3015 before may not be withdrawn from the slot 3015, so that the first connecting member 202 and the second connecting member 301 may be stably maintained in the current connected state.

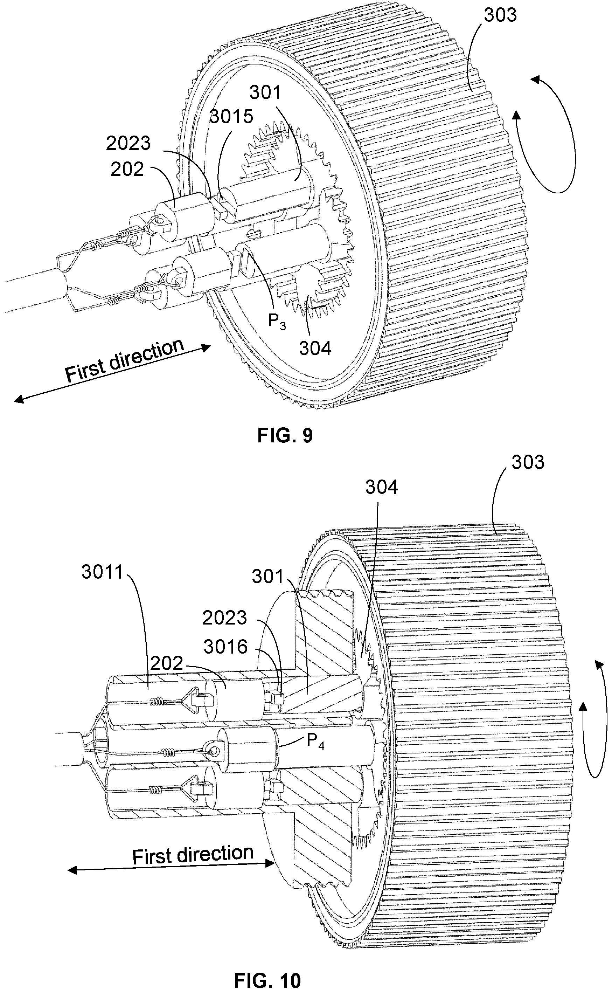

As another example, FIG. 9 is another schematic diagram illustrating a first connecting member and a second connecting member according to some embodiments of the present disclosure. FIG. 10 is another schematic diagram illustrating a first connecting member and a second connecting member according to some embodiments of the present disclosure. As shown in FIG. 9 and FIG. 10, the second connecting member 301 may rotate around an axis parallel to the first direction to switch the slot 3015 between the first position shown in FIG. 9 and the second position shown in FIG. 10. In the first state, the slot 3015 may be in the first position. In the second state, the slot 3015 may be in the second position. The first position and the second position may be different positions on a circumferential direction of the second connecting member 301 in the process of the second connecting member 301 rotating around an axis parallel to the first direction. For example, as shown in FIG. 9, in the first state, a point in the slot 3015 is located at position P3. As shown in FIG. 10, in the second state, the point in slot 3015 is located at position P4. When the slot 3015 is in the first position as shown in FIG. 9, the dimension of the slot 3015 along the second direction may be greater than the dimension of the insert block 2023 along the second direction, and the dimension of the slot 3015 along the third direction may be greater than the dimension of the insert block 2023 along the third direction, so that the insert block 2023 and the slot 3015 may be fitted and the insert block 2023 may be configured to be inserted into or withdrawn from the slot 3015 along the first direction.

In some embodiments, the first connecting member 202 or the second connecting member 301 may be further provided with a limiting hole 3016 (also be referred to as a second limiting hole 3016). The second limiting hole 3016 may be connected to the slot 3015. The dimension of the second limiting hole 3016 along the second direction may be greater than the dimension of the insert block 2023 along the second direction, and the dimension of the second limiting hole 3016 along the third direction may be greater than the dimension of the insert block 2023 along the third direction. In the first state, the insert block 2023 may be configured to be inserted into or withdrawn from the second limiting hole 3016 through the slot 3015 along the first direction.

When the first connecting member 202 needs to be connected to the second connecting member 301, the relative position between the insert block 2023 inserted into the second limiting hole 3016 and the slot 3015 may be adjusted, so that the width direction of the slot 3015 may be not parallel to the width direction of the insert block 2023, and the length direction of the slot 3015 may be not parallel to the length direction of the insert block 2023. The dimension of the slot 3015 along the second direction may be smaller than the dimension of the insert block 2023 along the second direction. At this time, since a structure of the slot 3015 along the second direction may limit the insert block 2023, the insert block 2023 may not be able to be withdrawn from the second limiting hole 3016 along the first direction. Alternatively, the dimension of the slot 3015 along the third direction may be smaller than the dimension of the insert block 2023 along the third direction. At this time, since the structure of the slot 3015 along the third direction may limit the insert block 2023, the insert block 2023 may not be able to be withdrawn from the second limiting hole 3016 along the first direction, thereby guaranteeing that the insert block 2023 inserted into the slot 3015 may not be withdrawn from the slot 3015, so that the first connecting member 202 and the second connecting member 301 may be stably maintained in the connected state.

When the first connecting member 202 and the second connecting member 301 in the connection need to be separated, the relative position of the insert block 2023 inserted into the second limiting hole 3016 and the slot 3015 may be adjusted again so that the width direction of the slot 3015 may be parallel to the width direction of the insert block 2023, and the length direction of the slot 3015 may be parallel to the length direction of the insert block 2023. At this time, the dimension of the width direction of the slot 3015 may be greater than the dimension of the width direction of the insert block 2023, and the dimension of the length direction of the slot 3015 may be greater than the dimension of the length direction of the insert block 2023. As a result, the dimension of the slot 3015 along the second direction may be greater than the dimension of the insert block 2023 along the second direction and the third direction, and the dimension of the slot 3015 along the third direction may be greater than the dimension of the insert block 2023 along the third direction. At this time, the insert block 2023 may be withdrawn from the second limiting hole 3016 in the first direction, thereby relieving the connected state of the first connecting member 202 and the second connecting member 301.

In some embodiments, since the dimension of the second limiting hole 3016 along one of the second direction and the third direction is greater than the dimension of the insert block 2023 in the one of the second direction and the third direction, when the insert block 2023 is located in the limiting hole, the slot 3015 and/or the insert block 2023 may rotate relative to each other around an axis parallel to the first direction to switch the slot 3015 and the insert block 2023 between the first state and the second state. For example, the slot 3015 may rotate around an axis parallel to the first direction to switch the slot 3015 and the insert block 2023 between the first state and the second state. As another example, the insert block 2023 may rotate around an axis parallel to the first direction to switch the slot 3015 and the insert block 2023 between the first state and the second state.

In some embodiments, the second limiting hole 3016 may be provided inside the first connecting member 202 or the second connecting member 301. As shown in FIG. 6A and FIG. 6B , the second limiting hole 3016 may be provided inside the second connecting member 301.

In some embodiments, the second limiting hole 3016 may be provided through at least one side of the first connecting member 202 or the second connecting member 301 along the second direction.

FIG. 6A is a partially enlarged view of a connection between a first connecting member and a second connecting member according to some embodiments of the present disclosure. FIG. 6B is another partially enlarged view of a connection between a first connecting member and a second connecting member according to some embodiments of the present disclosure. As shown in FIG. 6A and FIG. 6B, the second limiting hole 3016 may be provided through each of two sides of the second connecting member 301 along a second direction. In this way, the second limiting hole 3016 may be evasive to the insert block 2023 that extends into the slot 3015, so that a portion of an upper region of the insert block 2023 may extend outwardly along the second direction via the second limiting hole 3016. As a result, there may be no need to make a dimension of the second connecting member 301 too large to accommodate the insert block 2023, which is conducive to reducing the dimension of the second connecting member 301. The manufacturing process is also simpler, and there may be no need to set a cavity in the second connecting member 301 to accommodate the insert block 2023, and it is sufficient to directly dig through a wall surface of the second connecting member 301.

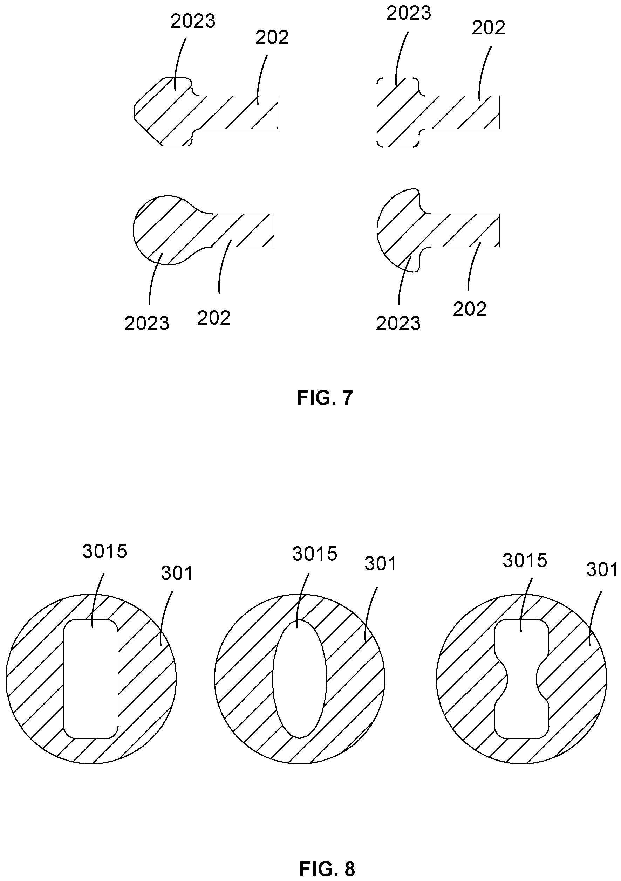

FIG. 7 is a schematic diagram illustrating a first connecting member according to some embodiments of the present disclosure. FIG. 8 is a schematic diagram illustrating a second connecting member according to some embodiments of the present disclosure. In some embodiments, as shown in FIG. 7, the cross-section of an end of the insert block 2023 along the first direction may be in various shapes (e.g., a cone, a square, a sphere, a hemisphere, etc.). As shown in FIG. 8, the cross-section of an inlet of the slot 3015 along the second direction may be in various shapes (e.g., a square, an ellipse, a dumb-bell shape, etc.). When the cross-section of the end of the insert block 2023 is in the cone along the first direction, the size of the insert block 2023 may change from small to large along a direction in which the insert block 2023 is inserted into the slot 3015, so that the insert block 2023 may be more easily aligned with the slot 3015 when beginning to be inserted into the slot 3015, reducing installation difficulty.

In some embodiments, the handle mechanism 300 may include a first gear ring 303. The handle mechanism 300 may further include a first gear 304 engaged with an inner wall of the first gear ring 303. The first gear 304 may be coaxially connected to the second connecting member 301. The first gear ring 303 may be configured to rotate around the first direction to drive the second connecting member 301 to rotate synchronously.

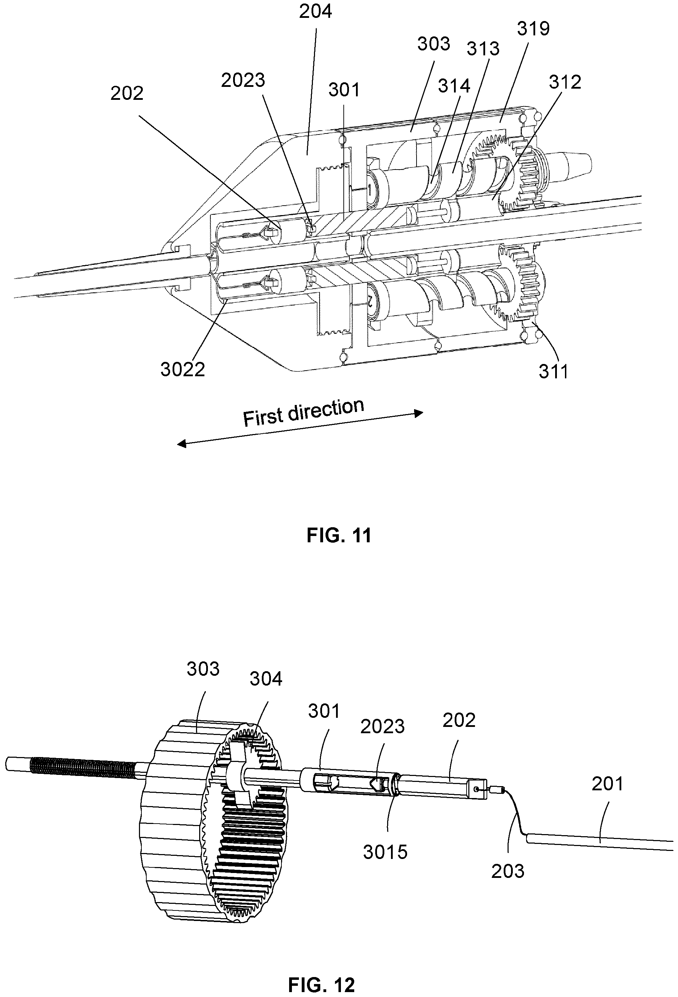

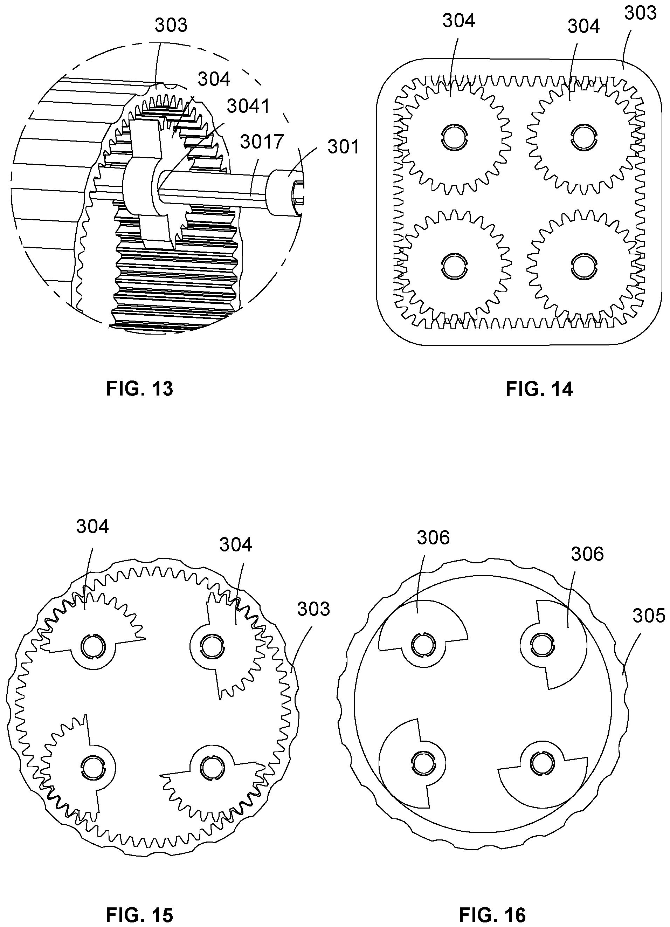

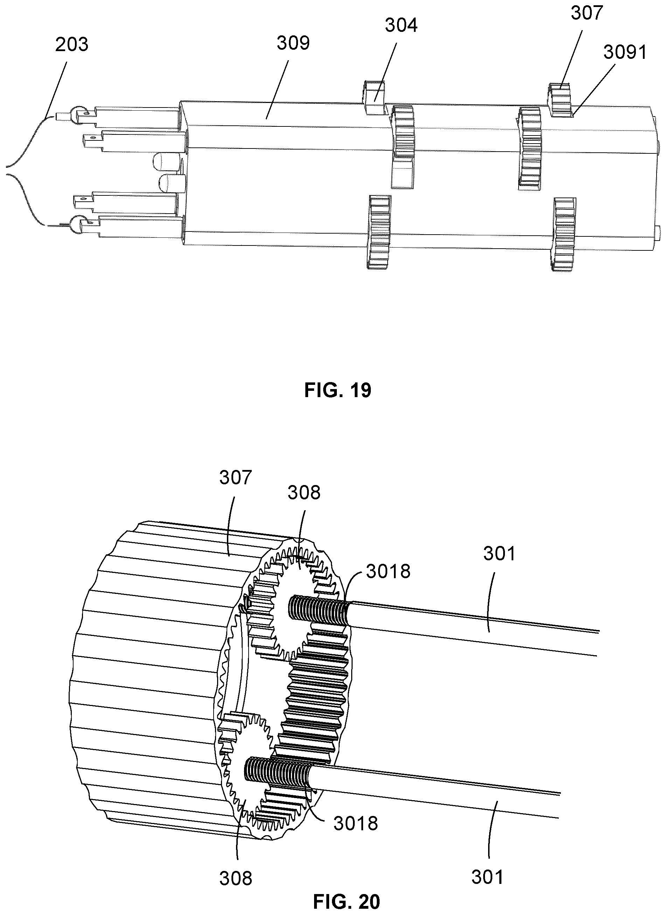

FIG. 12 is a schematic diagram illustrating a first gear ring, a first gear, and a second connecting member according to some embodiments of the present disclosure. FIG. 13 is a partially enlarged view of a connection of a first gear ring, a first gear, and a second connecting member according to some embodiments of the present disclosure. FIG. 14 is a schematic diagram illustrating a first gear ring and a first gear according to some embodiments of the present disclosure. FIG. 15 is another schematic diagram illustrating a first gear ring and a first gear according to some embodiments of the present disclosure. As shown in FIG. 12 and FIG. 13, the first gear 304 may be sleeved outside the second connecting member 301, and the first gear 304 and the second connecting member 301 may rotate synchronously. When the first gear ring 303 rotates, the first gear 304 engaged with the first gear ring 303 may synchronously rotate, which may in turn drive the second connecting member 301 to synchronously rotate, so that the slot 3015 may switch between the first position and the second position. In some embodiments, the first gear 304 may be set as a full gear as shown in FIG. 14. In some embodiments, the first gear 304 may be set as a half gear as shown in FIG. 15. In some embodiments, the first gear ring 303 may be configured to have a gear in only a portion of the inner wall. In some embodiments, the first gear ring 303 may be configured to have the gear throughout the inner wall. FIG. 19 is a schematic diagram illustrating a first gear, a second gear, and a second base according to some embodiments of the present disclosure As shown in FIG. 19, the first gear 304 may be embedded in a second base 309 in the handle mechanism 300 to limit movement of the first gear 304 in the first direction. More descriptions regarding the second base 309 may be found below.

In some embodiments, as shown in FIG. 13, the inner wall of the first gear 304 may be provided with a protrusion 3041, and an outer wall of the second connecting member 301 may be provided with a recessed portion 3017. The protrusion 3041 may be snapped into the recessed portion 3017, so that the second connecting member 301 may synchronously rotate along with the first gear 304 when the first gear 304 rotates. Positions of the protrusion 3041 and the recessed portion 3017 may be interchangeable, i.e., the recessed portion 3017 may be provided on the inner wall of the first gear 304, and the protrusion 3041 may be provided on the outer wall of the second connecting member 301. Alternatively, the first gear 304 and the second connecting member 301 may be integrally molded as a whole.

In some embodiments, the first gear ring 303 and the first gear 304 may also be provided in the conduit mechanism 200. Accordingly, the conduit mechanism 200 may include the first gear ring 303 and the first gear 304 engaged with the inner wall of the first gear ring 303. The first gear 304 may be coaxially connected to the first connecting member 202. The first gear ring 303 may be configured to rotate around the first direction to drive the first connecting member 202 to rotate synchronously, so that the insert block 2023 may switch between a first position and a second position.

In some embodiments, the handle mechanism 300 may include a first friction wheel 305 that is hollow inside and a second friction wheel 306 abutting an inner wall of the first friction wheel 305. The second friction wheel 306 may be coaxially connected to the second connecting member 301, and the first friction wheel 305 may be configured to rotate around the first direction to drive the second connecting member 301 to rotate synchronously.

The second friction wheel 306 and the second connecting member 301 may be connected in the same manner as the first gear 304 and the second connecting member 301 in the embodiments, which may not be repeated herein. FIG. 16 is a schematic diagram illustrating a first friction wheel and a second friction wheel according to some embodiments of the present disclosure. As shown in FIG. 16, an outer peripheral surface of the second friction wheel 306 may abut against an inner peripheral surface of the first friction wheel 305. When the first friction wheel 305 rotates around the first direction, the second friction wheel 306 may rotate synchronously accordingly, thereby driving the second connecting member 301 to rotate synchronously, so that the slot 3015 may switch between the first position and the second position.

In some embodiments, the first friction wheel 305 and the second friction wheel 306 may also be provided in the conduit mechanism 200. Accordingly, the conduit mechanism 200 may include the first friction wheel 305 that is hollow inside and the second friction wheel 306 abutting against the inner wall of the first friction wheel 305. The second friction wheel 306 may be coaxially connected to the first connecting member 202, and the first friction wheel 305 may be configured to rotate around the first direction to drive the first connecting member 202 to rotate synchronously, so that the insert block 2023 may switch between a first position and a second position.

As described above, the ultrasound probe 10 may include the multiple groups of connecting members each group of which includes a first connecting member 202 and a second connecting member 301 that are detachably connected.

In some embodiments, the first connecting members 202 and the second connecting members 301 in different groups may be detachably connected in the same manner. For example, in a connected state, the first connecting member 202 and the second connecting member 301 that are detachably connected in each group of the multiple groups of connecting members may be fitted together via a plug connection. That is, in the connection state, each insert block 2023 may be inserted into the slot 3015 corresponding to the insert block 2023 and may not be withdrawn from the slot 3015 to achieve a stable connection. Accordingly, the first gear 304 engaged with the inner wall of the first gear ring 303 may be sleeved outside each second connecting member 301, or the second friction wheel 306 engaged with the inner wall of the first friction wheel 305 may be sleeved outside each second connecting member 301. In the embodiments, providing the multiple groups of first connecting members 202 and second connecting members 301 in a fitted manner may improve the connection strength and connection stability of the conduit mechanism 200 and the handle mechanism 300 in the connected state, so that the conduit mechanism 200 and the handle mechanism 300 are not easy to be separated.

In some embodiments, the first connecting members 202 and the second connecting members 301 of the multiple groups of connecting members may be detachably connected in different manners. For example, the ultrasound probe 10 may include two groups of connecting members. The first connecting member 202 and the second connecting member 301 that are detachably connected in one group of connecting members may be fitted together via a plug connection. The first connecting member 202 and the second connecting member 301 that are detachably connected in the other group of connecting members may be fitted together via a thread connection.

By providing the first connecting member 202 and the second connecting member 301 as described in the above embodiment of the present disclosure, the first connecting member 202 may be detachably connected with the second connecting member 301. Furthermore, the conduit mechanism 200 and the handle mechanism 300 may be detachably connected, so that the conduit mechanism 200 may be replaced.

FIG. 11 is a schematic diagram illustrating some components of a conduit mechanism and a handle mechanism according to some embodiments of the present disclosure. As shown in FIG. 11, the conduit mechanism 200 may include a first housing 204. The handle mechanism 300 may further include a second housing 302. At least one first connecting member 202 may be disposed in the first housing 204. At least one second connecting member 301 may be disposed inside a connecting tube 3022 of the second housing 302. The cross-sectional shape of the first connecting member 202 perpendicular to a first direction may be adapted to a shape of a hollow portion of the connecting tube 3022 (e.g., as shown in FIG. 11, both the cross-sectional shape of the at least one first connecting member 202 perpendicular to the first direction and the shape of the hollow portion of the connecting tube 3022 may be in the form of a "D" shape), so as to ensure that the at least one first connecting member 202 may enter an interior of the connecting tube 3022 at a fixed angle and be connected to the at least one second connecting member 301 provided inside the connecting tube 3022 of the second housing 302, and after each first connecting member 202 is connected to one second connecting member 301, the first housing 204 and the second housing 302 may be fitted together via the thread connection, thereby guaranteeing the stability of the connection of each group of connecting members.

As shown in FIG. 2 and FIG. 3, the conduit mechanism 200 may further include the first housing 204 and the first base 205. The first base 205 may be installed in the first housing 204. Each of the at least one first connecting member 202 may penetrate through the first base 205. One end of each of the at least one bend-adjusting wire 203 may be connected with one of the at least one first connecting member 202, and the other end of the each of the at least one bend-adjusting wire 203 may extend into the flexible conduit 201 after penetrating through the first housing 204 and may be connected with an inner wall of a distal end of the flexible conduit 201. The insert block 2023 at an end of each first connecting member 202 of the at least one first connecting member 202 may penetrate through the first housing 204 and be exposed. The handle mechanism 300 may include the second housing 302 and the second base 309. The second base 309 may be fixedly installed in the second housing 302. The second base 309 may penetrate through the second housing 302 and an end of the second base 309 near the conduit mechanism 200 may be exposed. Each second connecting member 301 of the at least one second connecting member 301 may penetrate through the second base 309, so that the slot 3015 at an end of the second connecting member 301 may be exposed. The second housing 302 may include a grip portion 3021 for an operator to grip. In the connected state, the first housing 204 and the second housing 302 may be fitted together via the thread connection. That is, in the connected state, the two housings may be connected together. At the same time, each of the at least one second connecting member 301 and one of the at least one first connecting member 202 inside the two housings may be fitted via a plug connection. In some embodiments, referring to FIG. 2 and FIG. 3, the second base 309 may be provided with a positioning post 3092. Accordingly, the first base 205 may be provided with a positioning slot. The positioning post 3092 may be inserted in the positioning slot to position the handle mechanism 300 and the conduit mechanism 200, so that the insert block 2023 may be aligned with the slot 3015, thereby facilitating rapid completion of the installation. In other embodiments, positions of the positioning slot and the positioning post 3092 may be interchanged.

In some embodiments of the present disclosure, each group of connecting members may be detachably connected, which may, on the one hand, ensure that the conduit mechanism 200 may be controlled by the handle mechanism 300 when in use, so as to realize the adjustment of the direction of the ultrasonic transducer 100 provided at the distal end of the conduit mechanism 200, on the other hand, the conduit mechanism 200 connected to the handle mechanism 300 may be replaced after use, so that the handle mechanism 300 may be used repeatedly, reducing the cost of use of the ultrasound probe 10 and reducing the waste of resources.

In some embodiments, the operator may control the conduit mechanism 200 through the handle mechanism 300 of the ultrasound probe 10 to realize the adjustment of the direction of the ultrasonic transducer 100 provided at the distal end of the conduit mechanism 200. More descriptions regarding the handle mechanism 300 and the conduit mechanism 200 being provided to realize the adjustment of the direction of the ultrasonic transducer 100 provided at the distal end of the conduit mechanism 200 may be found below.

In some embodiments, the handle mechanism 300 may include a drive member. A helical structure may be provided on at least one of the drive member or the at least one second connecting member 301. The second connecting member 301 may be drivingly connected to the drive member via the helical structure. In the connected state, the drive member may be configured to rotate around the first direction to drive, via the helical structure, the at least one second connecting member 301 to move along the first direction.

In some embodiments, the drive member and the second connecting member 301 may be provided in various ways to guarantee that the second connecting member 301 may be driven to move along the first direction via the drive member.

A manner of setting the drive member is illustrated as follows.

In some embodiments, the drive member may include a second gear ring 307. The handle mechanism 300 may further include at least one second gear 308 engaged with the inner wall of the second gear ring 307. Each of the at least one second gear 308 may be provided with a first threaded portion. The helical structure may include a second threaded portion 3018 provided on each of one or more of the at least one second connecting member 301. The second threaded portion 3018 of the at least one second connecting member 301 may be threadedly connected with the first threaded portion of the at least one second gear 308. In the connected state, the second gear ring 307 may be configured to rotate around the first direction to drive the at least one second connecting member 301 to move in the first direction via the first threaded portion and the second threaded portion 3018. In some embodiments, the inner wall of the second gear ring 307 may be engaged with one second gear 308. The second gear 308 may be threadedly connected with the second threaded portion 3018 of one second connecting member 301 via the first threaded portion. In the connection state, the second gear ring 307 may drive the second connecting member 301 to move along the first direction when rotating in the first direction.

In some embodiments, the inner wall of a second gear ring 307 may be engaged with a plurality of second gears 308. FIG. 18 is a schematic diagram illustrating a second gear ring, a second gear, and a second connecting member according to some embodiments of the present disclosure. FIG. 20 is another schematic diagram illustrating a second gear ring, a second gear, and a second connecting member according to some embodiments of the present disclosure. In some embodiments, as shown in FIG. 18 and FIG. 20, the handle mechanism 300 may include the second gear ring 307 and the plurality of second gears 308 engaged with the inner wall of the second gear ring 307. The plurality of second gears 308 may be provided with a first threaded portion (not shown in FIG. 18 and FIG. 20). The second threaded portions 3018 of the plurality of second connecting members 301 may be threadedly connected with the first threaded portions of the plurality of second gears 308. In the connected state, the second gear ring 307 may be configured to rotate around the first direction, thereby driving each second connecting member 301 to move along the first direction via the first threaded portion and the second threaded portion 3018 that are threadedly connected.

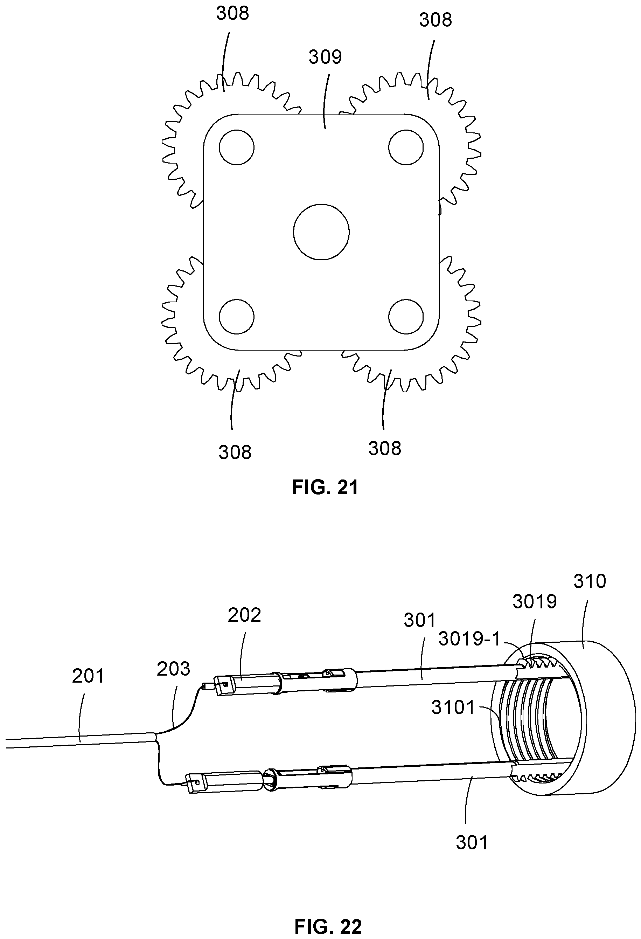

FIG. 21 is a schematic diagram illustrating a second gear according to some embodiments of the present disclosure. As shown in FIG. 18, FIG. 19, and FIG. 21, the second base 309 may be provided with a mounting groove 3091, and the plurality of second gears 308 may be mounted in the mounting groove 3091, such that the plurality of second gears 308 are restricted from moving along the first direction. When the second gear ring 307 rotates, the plurality of second gear 308 engaged with the second gear ring 307 may rotate synchronously, so that the plurality of second connecting members 301 threadedly connected to the plurality of second gears 308 may move along the first direction, thereby pulling or pushing the plurality of first connecting members 202 connected to the plurality of second connecting members 301 to move synchronously. When the plurality of first connecting members 202 move, the bend-adjusting wires 203 connected to the plurality of first connecting members 202 may also move synchronously, so as to pull or push the inner wall of the distal end of the flexible conduit 201, so that the distal end of the flexible conduit 201 may be bent.

It should be noted that, as shown in FIG. 19, the second base 309 may be equipped with the first gear 304 and the second gear 308. The second base 309 disposed in the handle mechanism 300 may restrict positions of the first gear 304 and the second gear 308, so that neither the first gear 304 nor the second gear 308 may move along the first direction, but may only rotate around the axis parallel to the first direction. On this basis, as shown in FIG. 1, FIG. 13, and FIG. 17, the first gear 304 may be driven to rotate around the axis parallel to the first direction through the first gear ring 303, thereby driving the first connecting member 202 or the second connecting member 301 to rotate around the axis parallel to the first direction, so that the first connecting member 202 and the second connecting member 301 may be detachably connected. In the process of using the ultrasound probe 10, the first connecting member 202 and the second connecting member 301 may be kept in the connected state. Therefore, after the first connecting member 202 is connected with the second connecting member 301, the first gear ring 303 and the first gear 304 may not continue to rotate around the axis parallel to the first direction, so as to avoid the first connecting member 202 separating from the second connecting member 301. In some embodiments, a helix angle of the second gear 308 may be greater than a helix angle of the first gear 304. In this way, when the second gear ring 307 rotates, the second gear 308 may be driven to rotate around the axis parallel to the first direction in the mounting groove 3091 of the second base 309. Since the second gear 308 may be threadedly connected with the second threaded portion 3018 of the second connecting member 301 through the first threaded portion, the second gear 308 may rotate around the axis parallel to the first direction, which may provide a driving force that drives the second connecting member 301 to rotate around the axis parallel to the first direction. However, since the second connecting member 301 is also connected with the first gear 304, and the helix angle of the second gear 308 is greater than the helix angle of the first gear 304, the driving force exerted on the second connecting member 301 when the second gear 308 rotates may be insufficient, and the first gear 304 and the first gear ring 303 engaged with the first gear 304 may not be driven to rotate. Therefore, when the second gear ring 307 rotates, the second connecting member 301 may not rotate around the axis parallel to the first direction. On this basis, the driving force may drive a threaded structure connected between the second connecting member 301 and the second gear 308 to be displaced relative to each other in the first direction. Since the second gear 308 may not move in the first direction, the driving force may drive the second connecting member 301 to move along the first direction. Therefore, when the second gear ring 307 rotates, the second connecting member 301 may be driven to move along the first direction, and the second connecting member 301 may not be driven to rotate around the axis parallel to the first direction, so that the first connecting member 202 and the second connecting member 301 may be kept in the connected state while the distal end of the flexible conduit 201 may be bent.

In some embodiments, the conduit mechanism 200 may include only one bend-adjusting wire 203. Accordingly, the at least one second gear 308 may include only one second gear 308 engaged with the inner wall of the second gear ring 307. The second gear ring 307 may rotate around the first direction to drive the second gear 308 to rotate synchronously, causing the second connecting member 301 threadedly connected to the second gear 308 to move along the first direction, which may drive the first connecting member 202 connected to the second connecting member 301 and the bend-adjusting wire 203 connected to the first connecting member 202 to synchronously move along the first direction, so that the second gear 308 may be drivingly connected to the bend-adjusting wire 203, thereby driving the distal end of the flexible conduit 201 to be bent, thereby adjusting the direction of the ultrasonic transducer 100 at the distal end of the flexible conduit 201.

In some embodiments, the conduit mechanism 200 may include at least one group of bend-adjusting wires 203, the at least one second gear 308 may include at least one group of second gears 308, and each of the at least one group of second gears 308 may include two second gears 308 engaged with the inner wall of the second gear ring 307. The two second gears 308 of each group of second gears 308 may be engaged with the inner wall of the same second gear ring 307.

In the connected state, each of the first bend-adjusting wire 203a and the second bend-adjusting wire 203b of each group of bend-adjusting wires 203 may be connected to one of two first connecting members 202. Each of the two first connecting members 202 may be connected to one of two second connecting members 301. Each of the two second connecting members 301 may be connected with one of the two second gears 308 of a group of second gears 308 through threads, and the two second connecting members 301 may have the threads in opposite directions. Therefore, when the second gear ring 307 rotates, the two bend-adjusting wires 203 each drivingly connected with the two second gears 308 may move in the opposite directions, so that bi-directional bending of the distal end of the flexible conduit 201 along one direction may be realized through cooperation of a group of the bend-adjusting wires 203.

In some embodiments, the two second gears 308 of different groups of second gears 308 may be engaged with the inner walls of different second gear rings 307 to control the bending of the distal end of the flexible conduit 201 in different directions through the different second gear rings 307. The setting method of another drive is described in the following section of this manual. Another manner of setting the drive member is illustrated as follows.

In some embodiments, the drive member may include a third gear ring 310. The at least one second connecting member 301 may be provided with a gear 3019. The helical structure may include a third threaded portion 3101 provided on an inner wall of the third gear ring 310 and a fourth threaded portion 3019-1 provided on the gear 3019. The fourth threaded portion 3019-1 provided on the gear 3019 may engage the third threaded portion 3101 provided on the inner wall of the third gear ring 310. In the connected state, the third gear ring 310 may be configured to rotate around the first direction to drive the gear 3019 to move along the first direction through the threaded fit between the third threaded portion 3101 provided on the inner wall of the third gear ring 310 and the fourth threaded portion 3019-1 provided on the gear 3019, thereby driving the second connecting member 301 provided with the gear 3019 to move along the first direction.

In some embodiments, the conduit mechanism 200 may include only one bend-adjusting wire 203. Accordingly, the only one second connecting member 301 may extend into the third gear ring 310. The gear 3019 of the second connecting member 301 may engage the inner wall of the third gear ring 310. The third gear ring 310 may rotate around the first direction to drive the gear 3019 to rotate synchronously, so that the second connecting member 301 provided with the gear 3019 may move along the first direction to drive the first connecting member 202 connected to the second connecting member 301 and the bend-adjusting wire 203 connected to the first connecting member 202 to move synchronously along the first direction, and the gear 3019 of the second connecting member 301 may be drivingly connected with the bend-adjusting wire 203.

In some embodiments, as shown in FIG. 22, the conduit mechanism 200 may include at least one group of bend-adjusting wires 203. In the connected state, two bend-adjusting wires 203 of each group of bend-adjusting wires 203 may be drivingly connected to gear 3019, respectively. The two gear 3019 may be engaged with different regions of an inner wall of one same third gear ring 310, and the fourth threaded portions 3019-1 of the two gears 3019 may have threads in opposite directions, so that when the second gear ring 307 rotates, the two bend-adjusting wires 203 each drivingly connected with the two gears 3019 may move in the opposite directions.

In some embodiments, the two gears 3019 corresponding to the different groups of bend-adjusting wires 203 may be engaged with inner walls of different third gear rings 310, so that the distal end of the flexible conduit 201 may be controlled to be bent in different directions through different gear rings.

As yet another manner of setting the drive member is illustrated as follows.

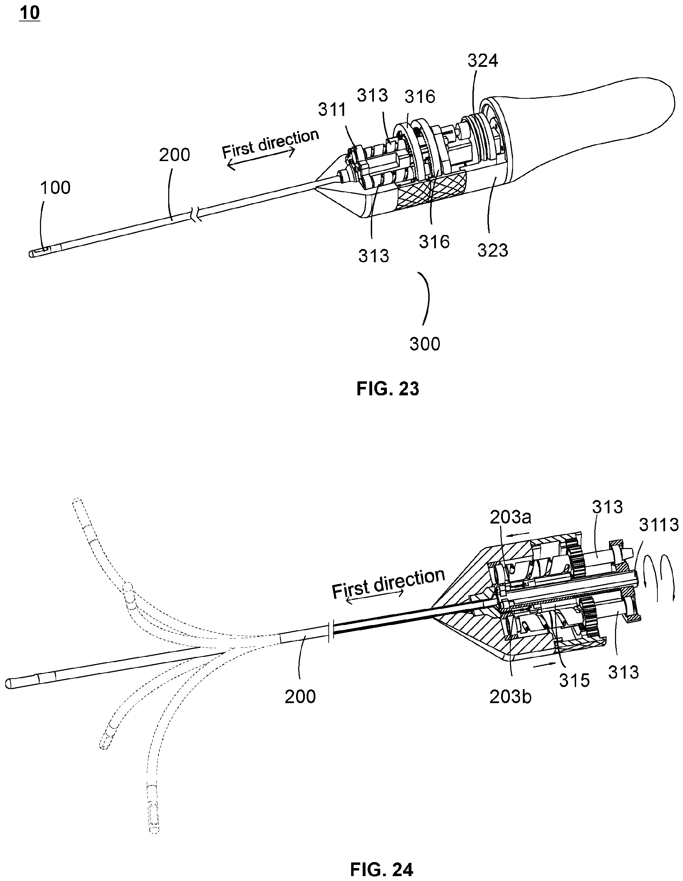

In some embodiments, as shown in FIG. 11 and FIG. 23, the handle mechanism 300 may further include a frame 311 and a limiting channel 312 extending along the first direction. The drive member may include at least one rotating rod 313 extending in the first direction and rotationally connected to the frame 311. The helical structure may include a helical portion 314 spirally arranged on an outer peripheral surface of each of the at least one rotating rod 313. The at least one second connecting member 301 may be provided in the limiting channel 312 and slide back and forth along the first direction in the limiting channel 312. The at least one second connecting member 301 may slidingly cooperate with the frame 311 in the first direction. The at least one second connecting member 301 may also cooperate with the helical portion 314 in a plug-in connection. In the connected state, the at least one rotating rod 313 may be configured to rotate around an axis parallel to the first direction to drive, via the helical portion 314, the at least one second connecting member 301 to move along the first direction, so that the direction of the distal end of the flexible conduit 201 may be adjusted, thereby adjusting the direction of the ultrasonic transducer 100. The ultrasound probe 10 described in the embodiment has a relatively small count of components and a relatively simpler overall structure so that the assembly difficulty and manufacturing cost are relatively low.

In the manner of setting the drive member, the first connecting member 202 may also be non-detachably connected with the second connecting member 301. When the first connecting member 202 is non-detachably connected with the second connecting member 301, the first connecting member 202 and the second connecting member 301 that are non-detachably connected may be called a sliding member 315. At least one sliding member 315 may be disposed in the handle mechanism 300. The at least one sliding member 315 may have all structural arrangements other than the structures used for detachable connection in the first connecting member 202 and the second connecting member 301. The at least one sliding member 315 may also have a connection relationship between the first connecting member 202 and other structures of the ultrasound probe 10 and a connection relationship between the second connecting member 301 and other structures of the ultrasound probe 10. For example, similar to the first connecting member 202, the at least one sliding member 315 may have a structure connected with the bend-adjusting wire 203 in the first connecting member 202 and may be connected with the bend-adjusting wire 203 through the structure. As another example, FIG. 24 is a schematic diagram illustrating a flexible conduit in different bending directions according to some embodiments of the present disclosure, as shown in FIG. 24, similar to the second connecting member 301, the at least one sliding member 315 may also be slidably matched to the frame 311 along the first direction. Each limiting channel 312 may be equipped with one sliding member 315, and the sliding member 315 may reciprocate along the first direction in the limiting channel 312. One of the at least one sliding member 315 may be plugged with the helical portion 314 of at least one rotating rod 313. Based on the structure, each of the at least one rotating rod 313 may be configured to rotate around an axis parallel to the first direction to drive the at least one sliding member 315 to move along the first direction through the helical portion 314, thereby driving at least one bend-adjusting wire 203 to move along the first direction, so as to adjust the direction of the distal end of the flexible conduit 201.

For ease of illustration, the sliding member 315 disposed in the handle mechanism 300 is illustrated as a basis as follows. However, it should be noted that a corresponding structure disposed on the at least one sliding member 315 can also be disposed on the second connecting member 301, and a connection relationship between the at least one sliding member 315 and other structures in the ultrasound probe 10 is also applicable to the second connecting member 301.

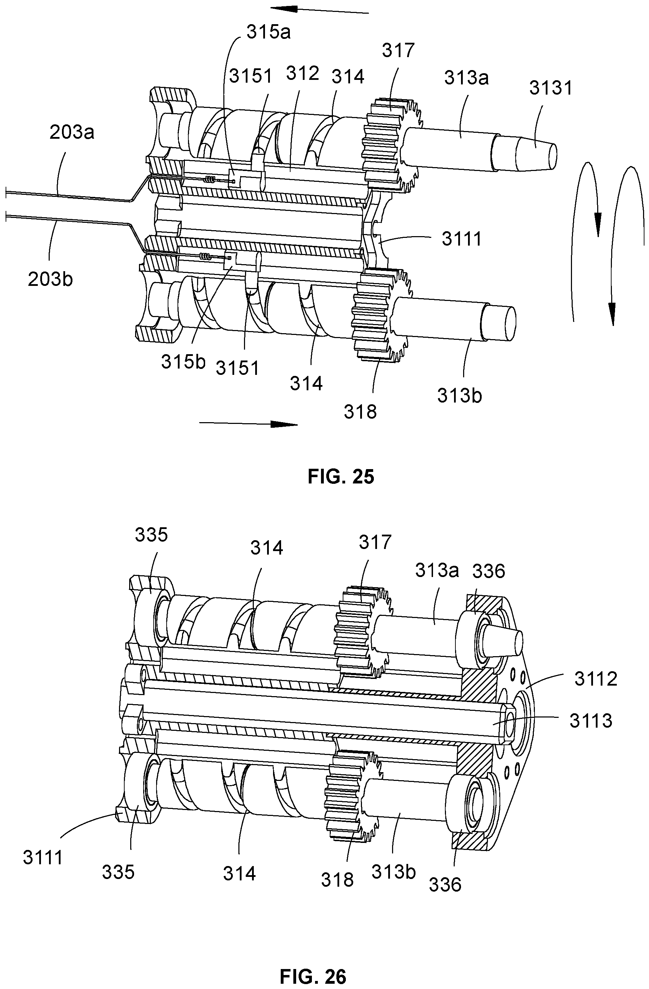

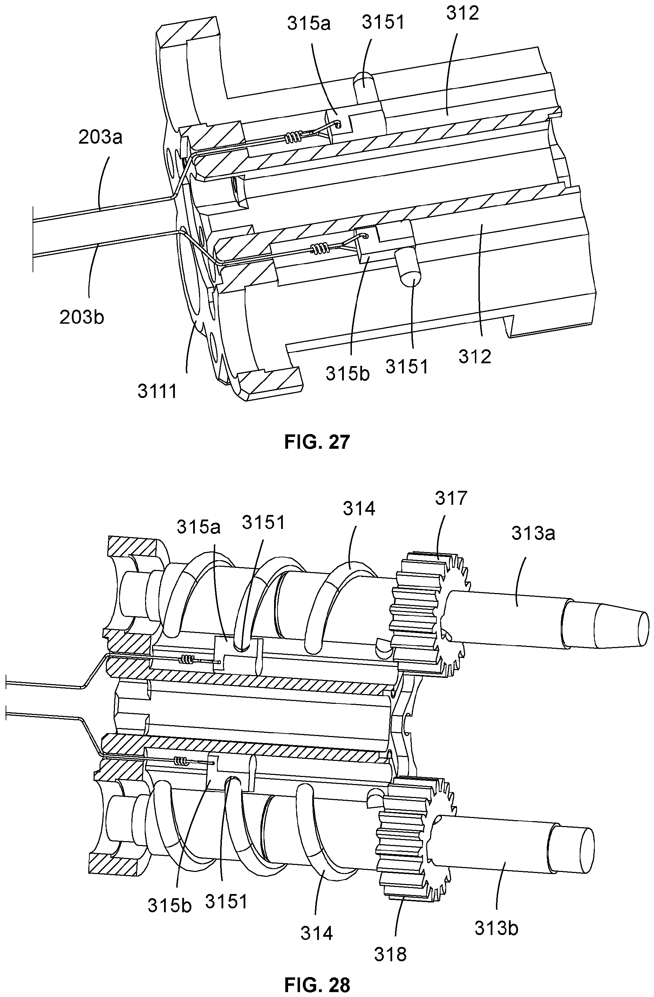

FIG. 25 is a schematic diagram illustrating a portion of a handle mechanism according to some embodiments of the present disclosure. FIG. 26 is a schematic diagram illustrating a frame, a first rotating rod, and a second rotating rod according to some embodiments of the present disclosure. FIG. 27 is a schematic diagram illustrating a frame, a sliding member, a first bend-adjusting wire, and a second bend-adjusting wire according to some embodiments of the present disclosure. In some embodiments, as shown in FIG. 25 to FIG. 27, the sliding member 315 may include a plug-in portion 3151. The helical portion 314 may be a helical groove recessed into an outer peripheral surface of the rotating rod 313. The plug-in portion 3151 may be a tab. The tab may be inserted into the helical groove. In this way, when the rotating rod 313 rotates around the axis parallel to the first direction, the helical groove of the rotating rod 313 may rotate accordingly, and the tab that is plugged with the helical groove may move along the first direction in the limiting channel 312.

Specifically, the proximal end of each bend-adjusting wire 203 may be connected with the sliding member 315. The plug-in portion 3151 is a tab with a rounded surface at the tip. The tab may be inserted into the helical groove, so that when the rotating rod 313 rotates around an axis parallel to the first direction, the groove wall of the helical groove may abut against the tab, thereby driving the sliding member 315 fitted with the rotating rod 313 to move in the first direction and realizing pulling or pushing the bend-adjusting wire 203.

FIG. 28 is another schematic diagram illustrating a portion of a handle mechanism according to some embodiments of the present disclosure. In some embodiments, as shown in FIG. 28, each of the at least one sliding member 315 may include the plug-in portion 3151. The helical portion 314 may be a helical projection projecting on an outer peripheral surface of the rotating rod 313. The plug-in portion 3151 may be a groove. The helical projection may be inserted into the groove. In this way, when the rotating rod 313 rotates around the first direction, the helical projection of the rotating rod 313 may rotate accordingly, and the groove fitted with the helical projection may move along the first direction in the limiting channel 312, thereby driving the sliding member 315 to move in the first direction.

In some embodiments, for the ultrasound probe 10 in an initial state, a position of the helical portion 314 that is plugged with the sliding member 315 may be a plug-in position. A distance between the plug-in position and the middle position of the helical portion 314 along the first direction may not exceed 10% of a length of the helical portion 314 along the first direction. "The length of the helical portion 314 along the first direction" refers to a spacing between a proximal end and a distal end of the helical portion 314 along the first direction. In this way, the sliding member 315 may have a more sufficient forward and backward travel when the rotating rod 313 rotates, so that a pulling or pushing amplitude of the bend-adjusting wire 203 may be larger, and a bend-adjusting amplitude may be larger.

Furthermore, in some embodiments, for the ultrasound probe 10 in an initial state, the plug-in position may coincide with the middle position of the helical portion 314 along the first direction. At this time, the forward and backward travel of the sliding member 315 may be maximized when the rotating rod 313 rotates, and the bend-adjusting magnitude may also be maximized.

In some embodiments, when the at least one bend-adjusting wire 203 is one bend-adjusting wire 203, accordingly, the handle mechanism 300 may include one rotating rod 313. In the connected state, the rotating rod 313 may rotate around an axis parallel to the first direction to drive the bend-adjusting wire 203 to move along the first direction via the helical portion 314, thereby realizing the adjustment of the direction of the ultrasonic transducer 100.

In some embodiments, the conduit mechanism 200 may include at least one group of bend-adjusting wires 203, and each group of the at least one group of bend-adjusting wires 203 may include a first bend-adjusting wire 203a and a second bend-adjusting wire 203b. The at least one rotating rod 313 may include at least one group of rotating rods 313. Each group of the at least one group of rotating rods 313 may include a first rotating rod 313a and a second rotating rod 313b. The at least one sliding member 315 may include at least one group of sliding members 315. The at least one group of sliding members 315 may include a first sliding member 315a and a second sliding member 315b.

For each group of bend-adjusting wires 203, each of two bend-adjusting wires 203 of the group of bend-adjusting wires 203 may be connected to each of two sliding members 315 of the group of sliding members 315. For example, the first bend-adjusting wire 203a may be connected to the first sliding member 315a, and the second bend-adjusting wire 203b may be connected to the second sliding member 315b. Each of the two sliding members 315 of the group of sliding members 315 may be fitted with each of two rotating rods 313 of the group of rotating rods 313. For example, the first sliding member 315a may be fitted with the first rotating rod 313a, and the second sliding member 315b may be fitted with the second rotating rod 313b.

In some embodiments, a count of groups of the at least one group of bend-adjusting wire 203 may be equal to 1. The count of groups of the at least one group of rotating rods 313 may be equal to 1. The count of groups of the at least one group of sliding members 315 may be equal to 1.

How the group of bend-adjusting wires 203 is configured to drive the first bend-adjusting wire 203a and the second bend-adjusting wire 203b to move in opposite directions along the first direction is illustrated as follows.

The first sliding member 315a connected to the first bend-adjusting wire 203a may cooperate with the helical portion 314 of the first rotating rod 313a in a plug-in connection to drive the first sliding member 315a to move in the first direction by rotating the first rotating rod 313a around an axis parallel to the first direction. The second sliding member 315b connected to the second bend-adjusting wire 203b may cooperate with the helical portion 314 of the second rotating rod 313b in a plug-in connection to drive the second sliding member 315b to move in the first direction by rotating the second rotating rod 313b around an axis parallel to the first direction. The first sliding member 315a and the second sliding member 315b may move in the opposite directions along the first direction. Accordingly, the first bend-adjusting wire 203a and the second bend-adjusting wire 203b may also move in the opposite directions along the first direction. In this way, the distal end of the flexible conduit 201 may be easier to be bent in a desired bending direction, with less difficulty in bending.

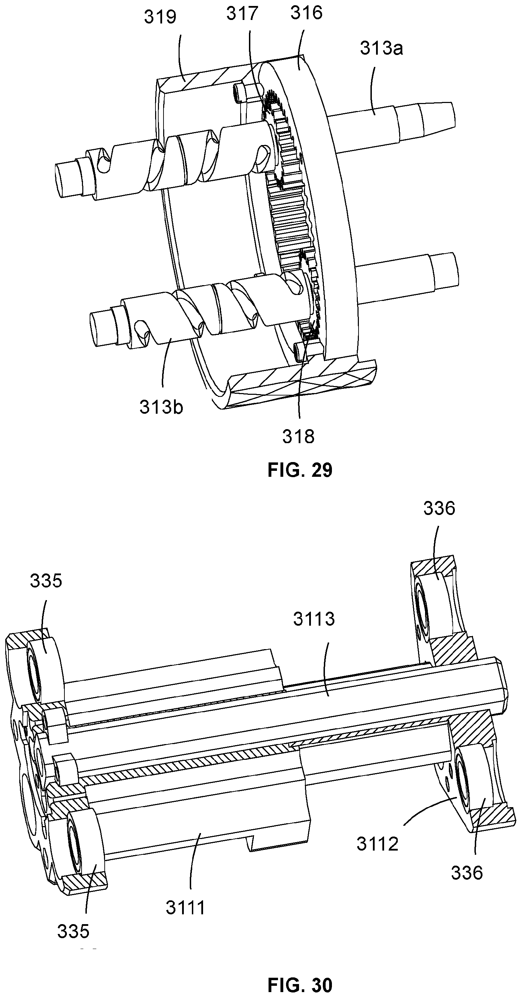

FIG. 30 is a schematic diagram illustrating a frame according to some embodiments of the present disclosure. Referring to FIG. 26, FIG. 27, and FIG. 30, in some embodiments, the frame 311 may include a first support 3111, a second support 3112, and a first central shaft 3113 that are fixedly connected. The first support 3111 and the second support 3112 may be fixedly connected via a threaded fastener. The first support 3111 and the second support 3112 may be fixed via snap connection. The first support 3111 and the second support 3112 may be integrally molded as a whole. The first central shaft 3113 may penetrate through the first support 3111 and the second support 3112 in turn and may be fixed to the first support 3111 and the second support 3112. The first support 3111 may be provided with two limiting channels 312 extending along the first direction, and the first sliding member 315a and the second sliding member 315b may be slidably installed in the two limiting channels 312, respectively. The frame 311 may be provided with two first bearings 335 and two second bearings 336. One end of each of the two rotating rods 313 may be rotationally connected to the first support 3111 via one first bearing 335. The other end of each of the two rotating rods 313 may be rotationally connected to the second support 3112 via one second bearing 336.

Referring to FIG. 25 to FIG. 27, in some embodiments, the two helical portions 314 may have opposite rotation directions. The two rotating rods 313 may be configured to rotate in a same direction around two axes parallel to the first direction, respectively.

Specifically, one of the two helical portions 314 may rotate in a left direction and the other of the two helical portions 314 may rotate in a right direction. Two rotating rod 313 may have a same rotation direction, so the helical portion 314 that rotate in the opposite directions may drive the two sliding members 315 to move in the opposite directions along the first direction, so that the first bend-adjusting wire 203a and the second bend-adjusting wire 203b may move in the opposite directions.

FIG. 29 is a schematic diagram illustrating a first rotating rod, a second rotating rod, a third gear, a fourth gear, and a fourth gear ring according to some embodiments of the present disclosure. Referring to FIG. 25, FIG. 26, and FIG. 29, in some embodiments, the handle mechanism 300 may include a fourth gear ring 316, a third gear 317 sleeved to the first rotating rod 313a, and a fourth gear 318 sleeved to the second rotating rod 313b. The third gear 317 and the fourth gear 318 may be both disposed in the fourth gear ring 316 and engaged with an inner wall of the fourth gear ring 316. The fourth gear ring 316 may be configured to rotate around the first direction to drive the first rotating rod 313a and the second rotating rod 313b to rotate in a same direction.

In some embodiments, the handle mechanism 300 may include a bend-adjusting knob 319 , the bend-adjusting knob 319 is sleeved on the outside of the fourth gear ring 316 . The operator may drive the first rotating rod 313a and the second rotating rod 313b to rotate by rotating the bend-adjusting knob 319 . Specifically, the bend-adjusting knob 319 may be sleeved outside the fourth gear ring 316, and the bend-adjusting knob 319 and the fourth gear ring 316 may be fixedly connected or integrally molded as a whole. Teeth may be provided in all regions of the inner wall of the fourth gear ring 316. Alternatively, the teeth may be provided in only some regions. The teeth may be engaged with the third gear 317 and the fourth gear 318. The third gear 317 may be connected to the first rotating rod 313a via a key or glue, so that the third gear 317 and the first rotating rod 313a may rotate synchronously. Similarly, the fourth gear 318 may be connected to the second rotating rod 313b via a key or glue, so that the fourth gear 318 and the second rotating rod 313b may rotate synchronously. Alternatively, the third gear 317 and the first rotating rod 313a may be directly integrally molded. Similarly, the fourth gear 318 and the second rotating rod 313b may be directly integrally molded. When the bend-adjusting knob 319 is turned, the fourth gear ring 316 may rotate synchronously with the bend-adjusting knob 319, thereby driving the third gear 317 and the fourth gear 318 engaged with the fourth gear ring 316 to rotate synchronously in a same direction, so that the first rotating rod 313a and the second rotating rod 313b may rotate in the same direction. In this way, the first rotating rod 313a and the second rotating rod 313b may be synchronously driven by merely turning the bend-adjusting knob 319 instead of driving the first rotating rod 313a and the second rotating rod 313b separately, which may simplify the structure and make the operation more convenient.

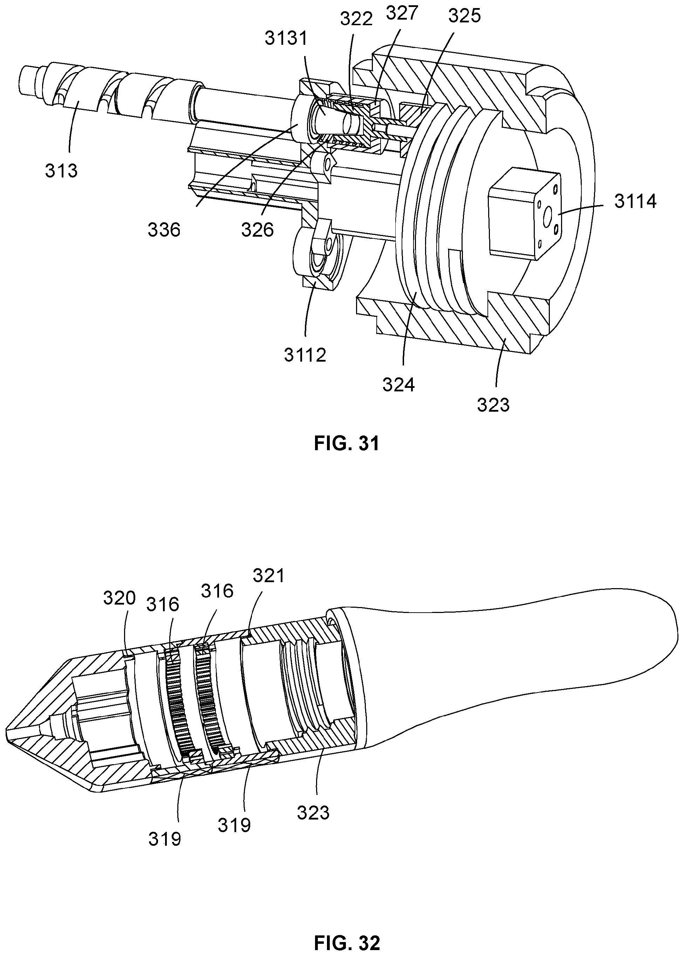

FIG. 32 is another schematic diagram illustrating a portion of a structure of a handle mechanism according to some embodiments of the present disclosure. Referring to FIG. 32, in some embodiments, a first pad 320 may be provided between the bend-adjusting knob 319 and a housing of the handle mechanism 300. The first pad 320 may be made of material (e.g., rubber, silicone, etc.) to increase friction force, so as to prevent the bend-adjusting knob 319 from rotating on itself when no external force is applied to rotate the bending knob 319, thereby the adjustment of the direction of the ultrasonic transducer 100 may be more stable and reliable.

In some embodiments, the two helical portions 314 may have a same rotation direction, and the two rotating rods 313 may be configured to rotate in opposite directions around two axes parallel to the first direction, respectively.

Specifically, the two helical portions 314 may both rotate in a left direction or in a right direction, and the two rotating rods 313 may rotate in opposite directions, so the two helical portions 314 having the same rotation direction may drive the two sliding members 315 respectively to move in the opposite directions along the first direction, so that the first bend-adjusting wire 203a and the second bend-adjusting wire 203b may move in the opposite directions.

Furthermore, in some embodiments, the handle mechanism 300 may include the third gear 317 sleeved to the first rotating rod 313a, and a fourth gear 318 sleeved to the second rotating rod 313b. The third gear 317 may be engaged with the fourth gear 318. The third gear 317 may be configured to rotate around the first direction to drive the two rotating rods 313 to rotate in opposite directions.

In some embodiments, the fourth gear ring 316 may be configured. The third gear 317 or the fourth gear 318 may be engaged with an inner wall of the fourth gear ring 316. The fourth gear ring 316 may be configured to rotate around the first direction to drive the first rotating rod 313a and the second rotating rod 313b to rotate in the opposite directions.

In some embodiments, the fourth gear ring 316 may also be omitted, and power may be directly applied to the third gear 317. Through rotation of the third gear 317, the fourth gear 318 engaged with the third gear 317 may be driven to rotate in the opposite directions, so that the first rotating rod 313a and the second rotating rod 313b may rotate in the opposite directions.

In some embodiments, the at least one bend-adjusting wire 203 may also include a plurality of groups of bend-adjusting wires 203. The at least one rotating rod 313 may include a plurality of groups of rotating rods 313. The at least one sliding member 315 may include a plurality of groups of sliding members 315 and a plurality of components (e.g., a plurality of fourth gear rings 316 along the first direction) controlling the bend-adjusting wires 203. Lines connecting distal ends of the plurality of groups of bend-adjusting wires 203 may intersect at a point (e.g., a center of the metal ring) to achieve bending of the distal end of the flexible conduit 201 in more directions.

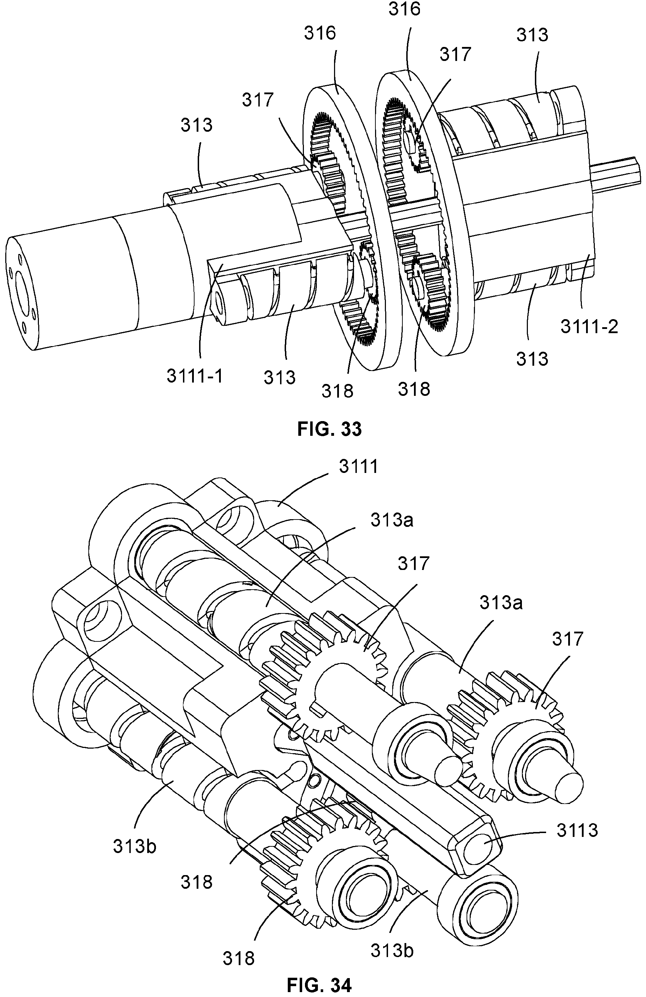

FIG. 34 is another schematic diagram illustrating a portion of a structure of a handle mechanism according to some embodiments of the present disclosure. For example, as shown in FIG. 34, the ultrasound probe 10 may include two groups of rotating rods 313, two groups of bend-adjusting wires 203 (not shown), and two groups of sliding members 315 (not shown). At this time, two rotating rods 313 of each group of rotating rods 313 may be symmetrically disposed on two sides of the first central shaft 3113, and a connecting line between two rotating rods 313 of one group of rotating rods 313 may be perpendicular to a connecting line between two rotating rods 313 of the other group of rotating rods 313. In this way, space of the frame 311 may be more fully utilized, resulting in a more compact structure and occupying less space. As shown in FIG. 24, four bend-adjusting wires 203 of two groups of bend-adjusting wires 203 may be controlled by two groups of rotating rods 313, thereby controlling a distal end of the flexible conduit 201 to be bent in four directions, so that the ultrasonic transducer 100 may have more directions.

FIG. 33 is a schematic diagram illustrating a rotating rod, a first support, a third gear, a fourth gear, and a fourth gear ring according to some embodiments of the present disclosure. As shown in FIG. 33, in other embodiments, the first support 3111 may include a first split seat 3111-1 and a second split seat 3111-2 provided in a split. The two groups of rotating rods 313 may be installed to the first split seat 3111-1 and the second split seat 3111-2, respectively.

In some embodiments, the handle mechanism 300 may include two bend-adjusting knob 319 arranged along the first direction, the two bend-adjusting knobs 319 are respectively used to control two groups of bend-adjusting wires 203. As shown in FIG. 32, a locking knob 323 may be disposed at the distal end of the two bend-adjusting knobs 319 along the first direction. A second spacer 321 may also be disposed between the bending knob 319 closer to the locking knob 323 and the locking knob 323..

In some embodiments, when a count of the at least one group of bend-adjusting wires 203 is greater than 1, structures of the handle mechanism 300 that drive each of the groups of bend-adjusting wires 203 may be the same or different. For example, the conduit mechanism 200 may include two groups of bend-adjusting wires 203. The two groups of bend-adjusting wires 203 may be drivingly connected to two groups of rotating rods 313. The two groups of rotating rods 313 may rotate around the axis parallel to the first direction to respectively control the two groups of bend-adjusting wires 203 to move along the first direction. As another example, the conduit mechanism 200 may include two groups of bend-adjusting wires 203. One group of bend-adjusting wires 203 may be drivingly connected to one group of rotating rods 313, and the one group of rotating rods 313 may rotate around the axis parallel to the first direction to control the group of bend-adjusting wires 203 to move along the first direction. The other group of bend-adjusting wires 203 may be drivingly connected to the other group of second gears 308, and the group of second gears 308 may rotate around the axis parallel to the first direction to control the group of bend-adjusting wires 203 to move along the first direction.

In some embodiments, the handle mechanism 300 may also include a locking assembly. The locking assembly may lock the rotating rod 313 to ensure the stability of the rotating rod 313, and avoid a change of direction of the ultrasonic transducer 100 caused by accidental rotation or self-rotation of the rotating rod.

FIG. 31 is a schematic diagram illustrating a locking assembly according to some embodiments of the present disclosure. Referring to FIG. 25, FIG. 26, and FIG. 31, in some embodiments, the locking assembly may include a locking sleeve 322. The rotating rod 313 may include a locking portion 3131. The locking sleeve 322 may be configured to move in a first direction to enable switching between a locked state and an unlocked state. In the locked state, the locking sleeve 322 may clamp the locking portion 3131, and in the unlocked state, the locking sleeve 322 may be separated from the locking portion 3131.

For example, an end of the first rotating rod 313a may form the locking portion 3131. The locking sleeve 322 may be configured to approach the locking portion 3131 along the first direction, so that the locking portion 3131 may be inserted into the locking sleeve 322 and clamped to inhibit rotation of the rotating rod 313, thereby inhibiting rotation of the third gear 317 connected to the first rotating rod 313a, so that the fourth gear ring 316 engaged with the third gear 317 may not rotate, the fourth gear 318 engaged with the fourth gear ring 316 may not rotate, and the second rotating rod 313b connected to the fourth gear 318 may not rotate. In this way, the rotation of the first rotating rod 313a and the second rotating rod 313b may be locked, so that the first bend-adjusting wire 203a and the second bend-adjusting wire 203b may be stably maintained in current positions. The ultrasonic transducer 100 may also be stably maintained in a current direction. Therefore, after the direction of the ultrasonic transducer 100 is adjusted, the locked state may be switched to. The unlocked state may be switched to when the direction of the ultrasonic transducer 100 needs to be adjusted again.

Referring to FIG. 25, FIG. 26, and FIG. 31, in some embodiments, the locking assembly may include the locking knob 323 and a push member 324. The push member 324 may slidingly cooperate with the frame 311 in the first direction, and the push member 324 may be threadedly connected to the locking knob 323. The locking knob 323 may be configured to rotate in the first direction to cause the push member 324 to push the locking sleeve 322 in the first direction close to the locking portion 3131 until the locking sleeve 322 is sleeved and clamped against an exterior of the locking portion 3131.

Specifically, the locking knob 323 may be sleeved to the exterior of the push member 324 and the locking knob 323 and the push member 324 may be threadedly connected. The frame 311 may further include a second central shaft 3114. The second central shaft 3114 may be fixedly connected to one side of the second support 3112 that deviates from the first support 3111. The push member 324 may slidingly cooperate with the second central shaft 3114 in the first direction. The second central shaft 3114 may be a square shaft, and the push member 324 may be sleeved to the exterior of the second central shaft 3114, so that rotation of the push member 324 around the first direction may be inhibited, and the push member 324 may move in the first direction when the locking knob 323 rotates around the first direction. The locking sleeve 322 may be fixedly connected with a pressure block 325. When the locking knob 323 rotates around the first direction, the push member 324 may be close to the pressure block 325 in the first direction until the push member 324 abuts against the pressure block 325, the pressure block 325 may be pushed. The locking sleeve 322 connected to the pressure block 325 may be pushed to be gradually close to the locking portion 3131, so that the locking portion 3131 may extend into the locking sleeve 322 and be clamped and locked by the locking sleeve 322.

In some embodiments, the first support 3111, the second support 3112, the first central shaft 3113, and the second central shaft 3114 of the frame 311 may be integrally molded as a whole.

In other embodiments, a driving structure may be directly provided. The push member 324 may be driven to be close to the pressure block 325 in the first direction through the driving structure.

Referring to FIG. 25, FIG. 26, and FIG. 31, in some embodiments, the locking sleeve 322 may have an inner cavity for housing the locking portion 3131. In a direction in which the locking sleeve 322 moves close to the locking portion 3131, a dimension of the inner cavity of the locking sleeve 322 and a dimension of the locking portion 3131 may gradually increase along the second direction. The second direction may be perpendicular to the first direction.

Specifically, one end of the locking sleeve 322 close to the locking portion 3131 may be provided with an opening for the locking portion 3131 to be inserted. The locking portion 3131 may be tapered. Accordingly, the inner cavity of the locking sleeve 322 may also be tapered. The dimensions of the locking portion 3131 and the inner cavity along the second direction may be radial dimensions. The inner cavity of the locking sleeve 322 and the locking portion 3131 may be configured to be in the tapered shape, so that the locking portion 3131 may be inserted into the locking sleeve 322, and the locking portion 3131 may be clamped and locked after the locking sleeve 322 continues to move. In other embodiments, the inner cavity of the locking portion 3131 and the locking sleeve 322 may also be configured to be in a prism shape.

In some embodiments, a cavity wall of the inner cavity for housing the locking portion 3131 of the locking sleeve 322 may be provided with a cushion made of material (e.g., rubber, silicone, etc.). Alternatively, an outer surface of the locking portion 3131 may be provided with the cushion made of material (e.g., rubber, silicone, etc.). As the locking sleeve 322 gradually moves toward the locking portion 3131, the locking portion 3131 may extend into the locking sleeve 322, and the cushion may abut against the locking portion 3131 to limit the rotation of the rotating rod 313. By providing the cushion, the friction force between the locking sleeve 322 and the locking portion 3131 may be increased, making the locked state more stable and reliable.

Referring to FIG. 25, FIG. 26, and FIG. 31, in some embodiments, the locking assembly may include an elastic member 326. The elastic member 326 may be connected to the locking sleeve 322. The elastic member 326 may be configured to drive the locking sleeve 322 to separate from the locking portion 3131 through a rebound force when the push member 324 moves in the first direction to a point of being disconnected with the locking sleeve 322.

Specifically, one side of the second support 3112 away from the first support 3111 may be provided with a mounting sleeve 327. The locking sleeve 322 may be mounted in the mounting sleeve 327 and may be configured to slide along the first direction in the mounting sleeve 327. The elastic member 326 may be sleeved to the exterior of the locking sleeve 322. One end of the elastic member 326 may be fixed to the second bearing 336 and the other end of the elastic member 326 may be fixed to the locking sleeve 322. When the locking sleeve 322 gradually moves toward the locking portion 3131, the elastic member 326 may be gradually compressed. When the locking knob 323 is turned in an opposite direction, and the push member 324 may be pushed in the opposite direction until the push member 324 separates from the pressure block 325, the push member 324 may be disconnected from the locking sleeve 322. Driven by the rebound force of the elastic member 326, the locking sleeve 322 may move in the opposite direction to reset, so that the locking portion 3131 may be disengaged from the locking sleeve 322 and no longer clamped and locked by the locking sleeve 322 to quickly switch to the unlocked state. In this way, automatic unlocking may be realized by the rebound force of the elastic member 326 by merely turning the locking knob 323 in the opposite direction, which is more convenient to operate and more efficient to unlock.

In other embodiments, the push member 324 may be fixed to the locking sleeve 322 directly. The locking sleeve 322 may be unlocked directly by the push member 324 driving the locking sleeve 322 to move in the opposite direction and reset.

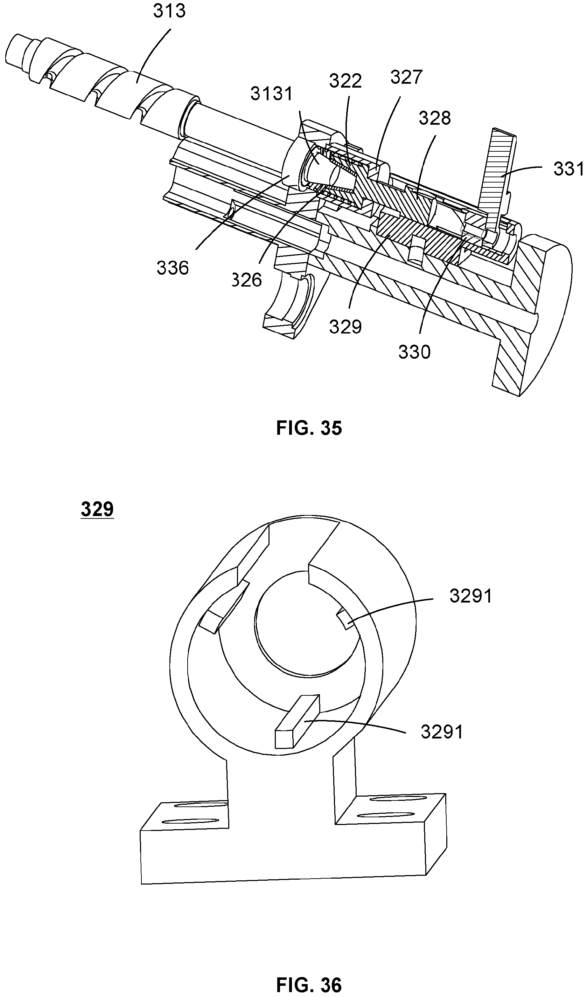

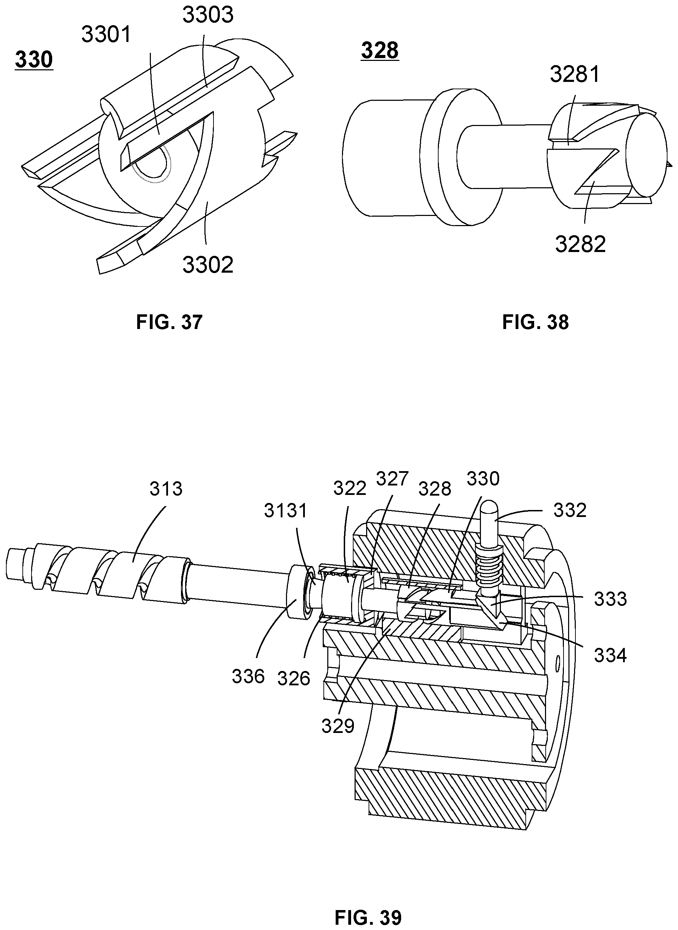

FIG. 35 is another schematic diagram illustrating a locking assembly according to some embodiments of the present disclosure. FIG. 36 is a schematic diagram illustrating a limiting sleeve according to some embodiments of the present disclosure. FIG. 37 is a schematic diagram illustrating a sawtooth member according to some embodiments of the present disclosure. FIG. 38 is a schematic diagram illustrating a moving member according to some embodiments of the present disclosure. Referring to FIG. 26, and from FIG. 35 to FIG. 38, in some embodiments, the locking assembly may include a moving member 328, a limiting sleeve 329, and the elastic member 326. The elastic member 326 and the moving member 328 may be connected to the locking sleeve 322. The limiting sleeve 329 may be connected to the frame 311. In the unlocked state, the moving member 328 may be configured to push the locking sleeve 322 to move in a first direction until the locking sleeve 322 is sleeved and clamped against an exterior of the locking portion 3131. The moving member 328 may resiliently abut against the limiting sleeve 329 under a rebound force of the elastic member 326. In the locked state, the moving member 328 may be configured to push the locking sleeve 322 to move in the first direction until the limiting sleeve 329 releases the support of the moving member 328, so that the locking sleeve 322 may be separated from the locking portion 3131 under the rebound force of the elastic member 326.

Specifically, the limiting sleeve 329 may be directly or indirectly fixed to the frame 311, for example, the limiting sleeve 329 may be fixed to the second support 3112 of the frame 311. In the embodiment, a connection structure between the locking sleeve 322, the locking portion 3131, the mounting sleeve 327, and the elastic member 326 is the same as that in the previous embodiment, with the difference being in the manner in which the locking sleeve 322 is driven to move. In the embodiment, the "ballpoint pen" structure is used for reference. In the unlocked state, the moving member 328 may be moved close to the locking sleeve 322 in the first direction, and the locking sleeve 322 may be pushed to move together to achieve the locking. The moving member 328 may resiliently abut against the limiting sleeve 329 under the rebound force of the elastic member 326, so as to limit the moving member 328 to a current position. At this time, the moving member 328 may move again along the first direction to release the abutting of the limiting sleeve 329 against the moving member 328. The locking sleeve 322 may move away from the locking portion 3131 under the rebound force of the elastic member 326 and may be separated from the locking portion 3131. In this way, the switching of unlocking or locking may be realized by merely moving the moving member 328 in the direction close to the locking sleeve 322, which is relatively easy to operate.

Referring to FIG. 35 to FIG. 38, in some embodiments, the locking assembly may include a sawtooth member 330. The moving member 328 and the locking sleeve 322 may be fixedly connected. The sawtooth member 330 may be disposed on one side of the moving member 328 that deviates from the locking sleeve 322. The limiting sleeve 329 may be sleeved outside of the moving member 328 and the sawtooth member 330. An inner wall of the limiting sleeve 329 may be provided with a convex rib 3291 extending in the first direction. The sawtooth member 330 may include a sliding block 3301 that extends in the first direction, a sawtooth portion 3302 that is integrated with the sliding block 3301 and is serrated, and a notched groove 3303 that extends in the first direction. An outer peripheral surface of the moving member 328 may be provided with a first limiting groove 3281 that extends in the first direction and is penetrating and a serrated second limiting groove 3282 connected to the first limiting groove 3281. The convex rib 3291 may be snapped in the notched groove 3303, so that the sawtooth member 330 may slide in the first direction relative to the limiting sleeve 329. In a first state, the convex rib 3291 may be snapped in the notched groove 3303 and the first limiting groove 3281. When the sawtooth member 330 approaches the locking sleeve 322 in the first direction, the moving member 328 may be pushed by the sawtooth member 330 to approach the limiting sleeve 329, so that the convex rib 3291 may exit the first limiting groove 3281. At the same time, the locking sleeve 322 may approach and clamp the locking portion 3131. A beveled plane of the sawtooth portion 3302 may cooperate with a beveled plane of the groove wall of the second limiting groove 3282, so that the moving member 328 may rotate around an axis parallel to the first direction until the convex rib 3291 extends into the second limiting groove 3282. With the rebound force of the elastic member 326, the groove wall of the second limiting groove 3282 may abut against an end of the convex rib 3291 to limit the moving member 328 and the locking sleeve 322 to current positions, so that the locking sleeve 322 may stably clamp the locking portion 3131. In a second state, the sawtooth member 330 may be again moved close to the locking sleeve 322 in the first direction, so that the convex rib 3291 may exit the second limiting groove 3282. The moving member 328 may rotate around an axis parallel to the first direction again under the action of the beveled plane of the sawtooth portion 3302 and the moving member 328 until the convex rib 3291 is simultaneously snapped into the adjacent notched groove 3303 and the first limiting groove 3281. At the same time, under the rebound force of the elastic member 326, the locking sleeve 322 may move away from the locking portion 3131 until the locking sleeve 322 is separated from the locking portion 3131.

In some embodiments, the handle mechanism 300 may further include a push rod 331. The push rod 331 and the sawtooth member 330 may be fixedly connected, and the unlocked state and the locked state may be switched by pushing the push rod 331.

FIG. 39 is another schematic diagram illustrating a locking assembly according to some embodiments of the present disclosure. Referring to FIG. 39, in other embodiments, a button 332 may also be configured. The button 332 may be connected with a first slant block 333. The sawtooth member 330 may be connected with a second slant block 334. A beveled plane of the first slant block 333 may cooperate with a beveled plane of the second slant block 334. A pushing direction of the button 332 may be perpendicular to the first direction. When the button 332 is pushed, the sawtooth member 330 may be moved along the first direction through a beveled plane of the first slant block 333 cooperating with a beveled plane of the second slant block 334, to realize the switching between the unlocked state and the locked state.

Having thus described the basic concepts, it may be rather apparent to those skilled in the art after reading this detailed disclosure that the foregoing detailed disclosure is intended to be presented by way of example only and is not limiting. Although not explicitly stated here, those skilled in the art may make various modifications, improvements and amendments to the present disclosure. These alterations, improvements, and modifications are intended to be suggested by this disclosure, and are within the spirit and scope of the exemplary embodiments of this disclosure.

In closing, it is to be understood that the embodiments of the present disclosure disclosed herein are illustrative of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. An ultrasound probe, comprising:
an ultrasonic transducer (100);
a conduit mechanism (200) including a flexible conduit (201) and at least one first connecting member (202), the flexible conduit (201) being connected to the ultrasonic transducer (100), and the at least one first connecting member (202) being connected to the flexible conduit (201); and
a handle mechanism (300) including at least one second connecting member (301), the handle mechanism (300) having a connected state or a disconnected state, in the connected state, the handle mechanism (300) being connected with the conduit mechanism (200) and in the disconnected state, the handle mechanism (300) being separated from the conduit mechanism (200), wherein
each of the at least one second connecting member (301) is detachably connected with one of the at least one first connecting member (202) to switch the handle mechanism (300) between the connected state and the disconnected state.

2. The ultrasound probe of claim 1, wherein the at least one second connecting member (301) and the at least one first connecting member (202) include a plurality of groups of first connecting members (202) and second connecting members (301), each group includes a first connecting member (202) and a second connecting member (301) that are detachably connected.

3. The ultrasound probe of claim 1 or claim 2, wherein the second connecting member (301) and the first connecting member (202) are configured to rotate relative to each other around an axis parallel to a first direction to enable the switching of the handle mechanism (300) between the connected state and the disconnected state, the first direction being parallel to a length direction of the handle mechanism (300).

4. The ultrasound probe of any one of claim 1 to claim 3, wherein one of the first connecting member (202) and the second connecting member (301) includes a slot (3015), the other one of the first connecting member (202) and the second connecting member (301) includes an insert block (2023), and a dimension of the slot (3015) along a second direction is smaller than a dimension of the slot (3015) along a third direction, the second direction, the third direction, and the first direction being perpendicular to each other;
at least one of the slot (3015) or the insert block (2023) is configured to rotate around an axis parallel to the first direction to switch the at least one of the slot (3015) or the insert block (2023) between a first state and a second state;
in the first state, the dimension of the slot (3015) along one of the second direction and the third direction is greater than a dimension of the insert block (2023) along the one of the second direction and the third direction, the insert block (2023) being configured to be inserted into or withdrawn from the slot (3015) along the first direction;
in the second state, the dimension of the slot (3015) in the second direction is smaller than the dimension of the insert block (2023) in the second direction, or the dimension of the slot (3015) in the third direction is smaller than the dimension of the insert block (2023) in the third direction to prevent the insert block (2023) from being withdrawn from the slot (3015) , preferably,
the slot (3015) is provided at a proximal end of the first connecting member (202) or a distal end of the second connecting member (301), and the first connecting member (202) or the second connecting member (301) is further provided with a limiting hole (3016), the limiting hole (3016) being connected to the slot (3015);
a dimension of the limiting hole (3016) along the second direction is greater than the dimension of the insert block (2023) along the second direction, and a dimension of the limiting hole (3016) along the third direction is greater than the dimension of the insert block (2023) along the third direction;
in the first state, the insert block (2023) is configured to be inserted into or withdrawn from the limiting hole (3016) through the slot (3015) along the first direction; and
when the insert block (2023) is located in the limiting hole (3016), at least one of the slot (3015) or the insert block (2023) rotates around an axis parallel to the first direction to switch the at least one of the slot (3015) or the insert block (2023) between the first state and the second state.

5. The ultrasound probe of claim 3, wherein
the handle mechanism (300) includes a first gear ring (303) and a first gear (304) engaged with an inner wall of the first gear ring (303), the first gear (304) is coaxially connected to the second connecting member (301), and the first gear ring (303) is configured to rotate around the first direction to drive the second connecting member (301) to rotate synchronously; or
the conduit mechanism (200) includes a first gear ring (303) and the first gear (304) engaged with an inner wall of the first gear ring (303), the first gear (304) is coaxially connected to the first connecting member (202), and the first gear ring (303) is configured to rotate around the first direction to drive the first connecting member (202) to rotate synchronously; or
the handle mechanism (300) includes a first friction wheel (305), and a second friction wheel (306) abutting an inner wall of the first friction wheel (305), the second friction wheel (306) is coaxially connected to the second connecting member (301), and the first friction wheel (305) is configured to rotate around the first direction to drive the second connecting member (301) to rotate synchronously; or
the conduit mechanism (200) includes a first friction wheel (305), and a second friction wheel (306) abutting the inner wall of the first friction wheel (305), the second friction wheel (306) is coaxially connected to the first connecting member (202), and the first friction wheel (305) is configured to rotate around the first direction to drive the first connecting member (202) to rotate synchronously.

6. The ultrasound probe of any one of claim 1 to claim 5, wherein the conduit mechanism (200) includes at least one bend-adjusting wire (203), a distal end of the at least one bend-adjusting wire (203) is connected to an inner wall of the distal end of the flexible conduit (201), and a proximal end of each bend-adjusting wire (203) of the at least one bend-adjusting wire (203) is connected to the first connecting member (202).

7. The ultrasound probe of any one of claim 1 to claim 5, wherein the handle mechanism (300) includes a drive member, a helical structure is provided on at least one of the drive member and the at least one second connecting member (301), and the at least one second connecting member (301) is drivingly connected to the drive member, and
in the connected state, the drive member is configured to rotate around the first direction to drive, via the helical structure, the at least one second connecting member (301) to move along the first direction, wherein the first direction is parallel to a length direction of the handle mechanism (300).

8. The ultrasound probe of claim 7, wherein the drive member includes a second gear ring (307), the handle mechanism (300) further includes at least one second gear (308) engaged with an inner wall of the second gear ring (307), each of the at least one second gear (308) being provided with a first threaded portion,
the helical structure includes a second threaded portion (3018) provided on each of the at least one second connecting member (301), the second threaded portion (3018) of each of the at least one second connecting member (301) being threadedly connected to the first threaded portion of the at least one second gear (308); and
in the connected state, the second gear ring (307) is configured to rotate around the first direction, thereby driving the at least one second connecting member (301) to move along the first direction via the first threaded portion and the second threaded portion (3018).

9. The ultrasound probe of claim 7, wherein the drive member includes a third gear ring (310), and the at least one second connecting member (301) is provided with a gear (3019),
the helical structure includes a third threaded portion (3101) provided on an inner wall of the third gear ring (310) and a fourth threaded portion (3019-1) provided on the gear (3019), the fourth threaded portion (3019-1) engaging the third threaded portion (3101), and
in the connected state, the third gear ring (310) is configured to rotate around an axis parallel to the first direction to drive the second connecting member (301) to move along the first direction, thereby driving the second connecting member (301) to move along the first direction via the gear (3019).

10. The ultrasound probe of claim 7, wherein the handle mechanism (300) further includes a frame (311) and a limiting channel (312) extending along the first direction, the drive member includes at least one rotating rod (313) extending in the first direction and rotationally connected to the frame (311), the helical structure includes a helical portion (314) spirally arranged on an outer peripheral surface of each of the at least one rotating rod (313),
the at least one second connecting member (301) is provided in the limiting channel (312) and slidingly cooperates with the frame (311) in the first direction, the at least one second connecting member (301) cooperates with the helical portion (314) in a plug-in connection, and
in the connected state, the at least one rotating rod (313) is configured to rotate around an axis parallel to the first direction to drive, via the helical portion (314), the at least one second connecting member (301) to move along the first direction.

11. The ultrasound probe of claim 10, wherein each of the at least one second connecting member (301) includes a plug-in portion (3151), the helical portion (314) is a helical groove recessed into the outer peripheral surface of the rotating rod (313), the plug-in portion (3151) includes a tab, the tab being inserted into the helical groove; or
each of the at least one second connecting member (301) includes a plug-in portion (3151), the helical portion (314) is a helical projection projecting on an outer peripheral surface of the second connecting member (301), and the plug-in portion (3151) includes a groove, the helical projection being inserted into the groove.

12. The ultrasound probe of claim 10, wherein the handle mechanism (300) includes a locking assembly, the locking assembly includes a locking sleeve (322), one of the at least one rotating rod (313) includes a locking portion (3131), and the locking sleeve (322) is configured to move in the first direction to enable switching between a locked state and an unlocked state;
in the locked state, the locking sleeve (322) clamps the locking portion (3131),
in the unlocked state, the locking sleeve (322) is separated from the locking portion (3131).

13. The ultrasound probe of claim 12, wherein the locking assembly further includes a locking knob (323) and a push member (324), the push member (324) slidingly cooperates with the frame (311) in the first direction, and the push member (324) is connected to the locking knob (323) through a thread, the locking knob (323) is configured to rotate in the first direction to cause the push member (324) to push the locking sleeve (322) to move close to the locking portion (3131) in the first direction until the locking sleeve (322) is sleeved and clamped against an exterior of the locking portion (3131).

14. The ultrasound probe of claim 12, wherein the locking sleeve (322) has an inner cavity for housing the locking portion (3131), and in a direction in which the locking sleeve (322) moves close to the locking portion (3131), a dimension of the inner cavity and a dimension of the locking portion (3131) gradually increase along a second direction, the second direction being perpendicular to the first direction; and/or
the locking assembly includes an elastic member (326), the elastic member (326) is connected to the locking sleeve (322), and the elastic member (326) is configured to drive the locking sleeve (322) to separate from the locking portion (3131) through a rebound force when the push member (324) moves in the first direction to a point of being disconnected with the locking sleeve (322).

15. The ultrasound probe of claim 12 wherein the locking assembly includes a moving member (328), a limiting sleeve (329), and an elastic member (326), the elastic member (326) and the moving member (328) are connected to the locking sleeve (322), and the limiting sleeve (329) is connected to the frame (311),
in the unlocked state, the moving member (328) is configured to push the locking sleeve (322) to move in the first direction until the locking sleeve (322) is sleeved and clamped against an exterior of the locking portion (3131), and the moving member (328) abuts against the limiting sleeve (329) under a rebound force of the elastic member (326);
in the locked state, the moving member (328) is configured to push the locking sleeve (322) to move in the first direction until the limiting sleeve (329) releases the support of the moving member (328), so that the locking sleeve (322) is separated from the locking portion (3131) under a rebound force of the elastic member (326).
